# EUROPEAN PATENT APPLICATION

(11) **EP 4 082 559 A1**
(43) Date of publication of application: **02.11.2022**
(21) Application number: 20904512.9
(22) Date of filing: 23.12.2020
(51) Int. Cl.: A61K 38/17, C07K 19/00, C07K 14/71, A61P 35/00, A61P 27/02

(54) **APPLICATION OF FUSION PROTEIN IN TREATING AGE-RELATED MACULAR DEGENERATION**

(30) Priority: 24.12.2019 CN 201911346311; 11.12.2020 CN 202011441025
(71) Applicant: Innovent Biologics (Suzhou) Co., Ltd., Suzhou, Jiangsu 215123 (CN)
(72) Inventor: LU, Shujie, Suzhou, Jiangsu 215123 (CN); QIAN, Lei, Suzhou, Jiangsu 215123 (CN)
(74) Representative: Teuten, Andrew John
(86) International application number: PCT/CN2020/138598
(87) International publication number: WO 2021/129658

(57) **Abstract**

The present invention relates to use of a fusion protein in the treatment of age-related macular degeneration. In particular, the present invention relates to use of a bispecific fusion protein inhibiting the activation of a complement pathway and a vascular endothelial growth factor (VEGF) pathway for the treatment of age-related macular degeneration. The present invention also relates to a pharmaceutical composition comprising the fusion protein and a method for treating age-related macular degeneration using the fusion protein.

## Description

### TECHNICAL FIELD

The present invention relates to use of a bispecific fusion protein inhibiting the activation of a complement pathway and a vascular endothelial growth factor (VEGF) pathway in the treatment of age-related macular degeneration. The present invention also relates to a pharmaceutical composition comprising the fusion protein and a method for treating age-related macular degeneration using the fusion protein.

### BACKGROUND

Age-related macular degeneration (AMD) is a chronic progressive disease of the retina in the central region. Age-related macular degeneration mostly occurs in people over 45 years old with the prevalence rate increasing with age, and is currently a severe disease causing blindness in the elderly. About 30 million people worldwide suffer from age-related macular degeneration, and about 0.5 million people go blind each year as a result. With the increasing economic development and aging of population in China, the incidence of AMD in China tends to increase year by year, and AMD has become the third leading cause of blindness in China.

AMD is primarily characterized by a decrease in the phagocytic and digestive capacity of the retinal pigment epithelium (RPE) to the outer segment disc membrane of visual cells, such that the incompletely digested disc membrane residual bodies are retained in the primary basal cell plasma and overflow out of the cells, where they are deposited on the Bruch's membrane to form drusen. Pigment epithelium, Bruch's membrane and visual cells at drusen are degenerated, proliferated or atrophied to different degrees. In the "age-related eye disease study" group of the National Eye Institute, AMD is divided into early AMD, intermediate AMD and late AMD, and early AMD is mainly characterized by drusen, and has high incidence and low blinding rate. As the disease progresses to the late stage, it seriously influences the visual acuity of patients, and can be classified as atrophic and exudative types according to the pathological characteristics. Exudative AMD, also known as wet AMD or neovascular AMD, is mainly characterized pathologically by increased release of vascular endothelial growth factors (VEGFs), immature neovascularization between pigment epithelial cells or photoreceptors at the center of the retina, i.e., choroidal neovascularization (CNV), subretinal rupture and hemorrhage, and the destruction of the retinal pigment epithelium and Bruch's membrane, with low incidence of disease and high blinding rate.

The pathological mechanisms of AMD have not been fully elucidated, and it is now generally accepted that angiogenesis induced by increased expression of VEGFs is major pathogenesis of wet AMD. In addition, inflammatory responses mediated by the complement gene variation and complement abnormal activation are also considered to be important pathogenesis of AMD. Activation of the complement system has a direct effect on RPE damage. In one aspect, it leads to RPE atrophy and induces the atrophy of cells at the center of the retina, thereby forming GA (atrophic AMD); in another aspect, it disrupts the blood-retinal barrier, and leads to inflammation, high expression of VEGFs and neovascularization, thereby forming neovascular AMD (exudative AMD).

Anti-complement drugs are mainly used for dry AMD accompanied by geographic atrophy, but their efficacy is not clear for wet AMD with higher blindness and may need to be combined with anti-VEGF drugs to show advantages. However, the existing combination therapy is performed by the intravitreal injection of an anti-complement drug and an anti-VEGF drug, and there are no dual antibody drugs comprising the anti-complement drug and the anti-VEGF drug. Since the intravitreal injection has certain risks, and patients have poor compliance when they are repeatedly administered for a long time, the combination therapy increases the risks of injection operation and imposes great psychological and economic burdens on the patients, so that the development of the medicament capable of inhibiting both a VEGF and a complement is potentially of clinical value. In addition, the complement activation plays an important role in the formation of geographic atrophy, and the fusion protein of both anti-complement and anti-VEGF drugs can be of significant clinical value for wet AMD patients accompanied by geographic atrophy.

WO2013082563A1 discloses a novel fusion protein inhibiting a VEGF pathway and a complement pathway. However, WO2013082563A1 only verified the activity of the fusion protein in animals, and studies on the efficacy of the fusion protein in human AMD diseases are still in progress. In addition, considering the complexity of AMD disorders in the eyes of humans, the limitations and specificities of the handling and drug volume of ocular administration, etc., there is still a need for in-depth research and improvement of methods and specific protocols for the use of such fusion proteins for human AMD diseases.

### SUMMARY

### I. Treatment Method

In a first aspect, the present invention relates to a method for preventing or treating age-related macular degeneration (AMD) in an individual, which comprises administering to the individual a fusion protein inhibiting a VEGF pathway and a complement pathway.

In an embodiment, the fusion protein comprises a sequence of SEQ ID NO: 1:

DTGRPFVEMYSEIPEIIHMTEGRELVIPCRVTSPNITVTLKKFPLDTLIPDGKKIIWDSRKGFIISNATYKEIG
LLTCEATVNGHLYKTNYLTHRQTNTIIDVVLSPSHGIELSVGEKLVLNCTARTELNVGIDFNWEYPSSKH
QHKKLVNRDLKTQSGSEMKKFLSTLTIDGVTRSDQGLYTCAASSGLMTKKNSTFVRVHEKDKTHTCPPC
PAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHY
TQKSLSLSPGKGGGGGGQCNAPEWLPFARPTNLTDEFEFPIGTYLKYECRPGYYGRPFSIICLKNSVWTGA
KDRCRRKSCRNPPDPVNGMVHVIKDIQFGSQIKYSCTKGYRLIGSSSATCIISGNTVIWDNETPICDRIPCG
LPPTITNGDFISTNRENFHYGSVVTYRCNPGSGGRKVFELVGEPSIYCTSNDDQVGIWSGPAPQCI (SEQ ID
NO: 1).

In a preferred embodiment, the sequence of the fusion protein is a sequence set forth in SEQ ID NO: 1. For convenience of expression, the fusion protein is referred to herein as IBI302 and the preparation method thereof is described in WO2013082563A1.

In an embodiment, the AMD is wet AMD.

In an embodiment, the wet AMD has one or more of the following symptoms and signs: visual acuity decrease, metamorphopsia, central scotoma, dyslexia, macular retinal swelling, fundus hemorrhage, neovascularization, scar fibrosis, geographic atrophy, and the like, or any combination thereof.

In an embodiment, the wet AMD has choroid neovascularization (CNV).

In an embodiment, the wet AMD is AMD having the following characteristics:
1) active CNV under the macular fovea secondary to wet AMD occurs in a study eye; and/or
2) central subfield thickness is ≥ 250 µm through optical coherence tomography (OCT) scanning.

In an embodiment, the individual is a human individual.

In a preferred embodiment, the individual is a human individual having the disease, symptoms, and/or characteristics as described in any of the above embodiments.

In a preferred embodiment, the individual is a human individual who is a wet AMD patient accompanied by geographic atrophy.

In a preferred embodiment, the individual suffers from wet AMD with the following characteristics:
1) active CNV under the macular fovea secondary to wet AMD occurs in a study eye; and/or
2) central subfield thickness is ≥ 250 µm through optical coherence tomography (OCT) scanning.

In a preferred embodiment, the treatment may achieve one or more of the following effects in the individual:
1) central subfield thickness is decreased from a baseline;
2) area of the choroidal neovascularization (CNV) is reduced;
3) best corrected visual acuity (BCVA) is improved from a baseline;
4) onset of the geographic atrophy in the wet AMD patient is lagged; and/or
5) onset of the geographic atrophy in the wet AMD patient is slowed.

In a preferred embodiment, the treatment may achieve one or more of the following effects in the individual:
1) best corrected visual acuity (BCVA) is improved; and/or
2) central subfield thickness is decreased; and/or
3) choroidal neovascularization (CNV) area is reduced.

In a preferred embodiment, the treatment may achieve one or more of the following effects in the individual:
1) best corrected visual acuity (BCVA), as compared to a baseline, is improved by at least 1 ETDRS letter, preferably by at least 5 ETDRS letters, more preferably by 5-35 ETDRS letters, for example, by 5-25 ETDRS letters, for example, by 5-10, 5-15, 5-20, 10-15, 10-20, 10-25, 15-20 or 15-25 letters, for example, by 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 letters; and/or
2) best corrected visual acuity (BCVA), as compared to a baseline, is improved by 5% or more, preferably by 10% or more, for example, by 10%-200%, preferably by 10%-150%, more preferably by 10%-100%, more preferably by 20%-100%, more preferably by 50%-100%, 60%-100%, 20%-80% or 30%-70%, more preferably by 50%-70%, more preferably by 60%-70%, for example, by 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190% or 200%, and/or
3) central subfield thickness (CST), as compared to a baseline, is decreased by 25 µm or more, for example, by 25-350 µm, preferably by 50-300 µm, more preferably by 50-200 µm, for example, by 25 µm, 50 µm, 75 µm, 100 µm, 125 µm, 150 µm, 175 µm, 200 µm, 225 µm, 250 µm, 275 µm, 300 µm, 325 µm or 350 µm; or is decreased by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%; and/or
4) area of the choroidal neovascularization (CNV), as compared to a baseline, is reduced by 0.1 mm² or more, for example, by 0.1-20 mm², preferably by 0.2-5 mm², more preferably by 0.3-2 mm², for example, by 0.1 mm², 0.2 mm², 0.3 mm², 0.4 mm², 0.5 mm², 0.6 mm², 0.7 mm², 0.8 mm², 0.9 mm², 1 mm², 1.1 mm², 1.2 mm², 1.3 mm², 1.4 mm², 1.5 mm², 1.6 mm², 1.7 mm², 1.8 mm², 1.9 mm², 2 mm², 2.1 mm², 2.2 mm², 2.3 mm², 2.4 mm², 2.5 mm², 2.6 mm², 2.7 mm², 2.8 mm², 2.9 mm², 3 mm², 3.1 mm², 3.2 mm², 3.3 mm², 3.4 mm², 3.5 mm², 3.6 mm², 3.7 mm², 3.8 mm², 3.9 mm², 4 mm², 4.1 mm², 4.2 mm², 4.3 mm², 4.4 mm², 4.5 mm², 4.6 mm², 4.7 mm², 4.8 mm², 4.9 mm², 5 mm², 5.5 mm², 6 mm², 6.5 mm², 7 mm², 7.5 mm², 8 mm², 8.5 mm², 9 mm², 9.5 mm², 10 mm², 10.5 mm², 11 mm², 11.5 mm², 12 mm², 12.5 mm², 13 mm², 13.5 mm², 14 mm², 14.5 mm², 15 mm², 15.5 mm², 16 mm², 16.5 mm², 17 mm², 17.5 mm², 18 mm², 18.5 mm², 19 mm², 19.5 mm² or 20 mm²; or is reduced by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

In some embodiments, the fusion protein of the present invention is formulated as a liquid pharmaceutical composition for administration. Useful carriers and solvents include water, Ringer's solution, phosphate buffered saline, isotonic sodium chloride solution and the like. In addition, sterile non-volatile oils may also be used as a solvent or suspending medium, where appropriate. For this purpose, any mixture of non-volatile mineral oils or non-mineral oils may be used, including synthetic monoglycerides or diglycerides. In addition, fatty acids such as oleic acid may also be used in the preparation of injections.

In some embodiments, the pharmaceutical composition comprising the fusion protein of the present invention is a solution for injection or a dry powder formulation. For example, the composition is a lyophilized powder, which may be formulated as an injection in a pharmaceutically acceptable liquid carrier. The pharmaceutically acceptable liquid carrier may be, for example, sterile water, Ringer's solution, phosphate buffered saline, isotonic sodium chloride solution and the like.

In some embodiments, the fusion protein of the present invention is administered topically.

In some embodiments, the fusion protein of the present invention is injected intravenously.

In some embodiments, the fusion protein of the present invention is administered intralesionally.

In some embodiments, the pharmaceutical composition comprising the fusion protein of the present invention is administered directly to an eye or ocular tissue. In some embodiments, the pharmaceutical composition is administered topically to an eye. In some embodiments, the pharmaceutical composition is administered by injection to an eye (intraocular injection) or a tissue associated with the eye. The composition may be administered, for example, by intraocular injection, periocular injection, subretinal injection, intravitreal injection, trans-septal injection, sub-scleral injection, intrachoroidal injection, intracameral injection, subconjunctival injection, subtenon injection, retrobulbar injection, peribulbar injection, or posterior juxtascleral delivery. The composition may also be administered, for example, to vitreous, optic nerve, aqueous humor, sclera, conjunctiva, the area between the sclera and conjunctiva, retinochoroidal tissue, macula, or other areas in or near the eye of the individual. Preferably, the composition is injected intravenously.

In some embodiments, the fusion protein of the present invention is administered at a frequency of three times a day, twice a day, once a day, once every two days, twice a week, once a week, once every two weeks, once every four weeks, once every five weeks, once every six weeks, once every seven weeks, once every eight weeks, once a month, once every two months, or longer. The fusion protein of the present invention may be administered in a cycle of one week, two weeks, three weeks, one month, two months, three months or longer, and the intervals between every two administration cycles may be the same or different. In a preferred embodiment, the fusion protein of the present invention is administered once or twice, preferably once, in each administration cycle, in the mode of administration of administering on the first day of each cycle.

In each of the above embodiments, a single dose of the fusion protein of the present invention may be selected from 0.01-10 mg/eye, preferably 0.05-8 mg/eye, more preferably 0.05-6 mg/eye, more preferably 0.5-5 mg/eye, more preferably 1-5 mg/eye, more preferably 2-5 mg/eye, for example, 0.5-2 mg/eye, 1-3 mg/eye, 2-4 mg/eye, 2-3 mg/eye or 3-5 mg/eye, for example, 0.05 mg/eye, 0.1 mg/eye, 0.15 mg/eye, 0.2 mg/eye, 0.25 mg/eye, 0.3 mg/eye, 0.35 mg/eye, 0.4 mg/eye, 0.45 mg/eye, 0.5 mg/eye, 0.55 mg/eye, 0.6 mg/eye, 0.65 mg/eye, 0.7 mg/eye, 0.75 mg/eye, 0.8 mg/eye, 0.85 mg/eye, 0.9 mg/eye, 0.95 mg/eye, 1 mg/eye, 1.1 mg/eye, 1.2 mg/eye, 1.3 mg/eye, 1.4 mg/eye, 1.5 mg/eye, 1.6 mg/eye, 1.7 mg/eye, 1.8 mg/eye, 1.9 mg/eye, 2 mg/eye, 2.1 mg/eye, 2.2 mg/eye, 2.3 mg/eye, 2.4 mg/eye, 2.5 mg/eye, 2.6 mg/eye, 2.7 mg/eye, 2.8 mg/eye, 2.9 mg/eye, 3 mg/eye, 3.1 mg/eye, 3.2 mg/eye, 3.3 mg/eye, 3.4 mg/eye, 3.5 mg/eye, 3.6 mg/eye, 3.7 mg/eye, 3.8 mg/eye, 3.9 mg/eye, 4 mg/eye, 4.1 mg/eye, 4.2 mg/eye, 4.3 mg/eye, 4.4 mg/eye, 4.5 mg/eye, 4.6 mg/eye, 4.7 mg/eye, 4.8 mg/eye, 4.9 mg/eye, 5 mg/eye, 5.1 mg/eye, 5.2 mg/eye, 5.3 mg/eye, 5.4 mg/eye, 5.5 mg/eye, 5.6 mg/eye, 5.7 mg/eye, 5.8 mg/eye, 5.9 mg/eye, 6 mg/eye, 6.1 mg/eye, 6.2 mg/eye, 6.3 mg/eye, 6.4 mg/eye, 6.5 mg/eye, 6.6 mg/eye, 6.7 mg/eye, 6.8 mg/eye, 6.9 mg/eye, 7 mg/eye, 7.1 mg/eye, 7.2 mg/eye, 7.3 mg/eye, 7.4 mg/eye, 7.5 mg/eye, 7.6 mg/eye, 7.7 mg/eye, 7.8 mg/eye, 7.9 mg/eye, 8 mg/eye, 8.5 mg/eye, 9 mg/eye, 9.5 mg/eye or 10 mg/eye, preferably 2 mg/eye or 4 mg/eye. The mode of administration of the dose is preferably directly administrating the pharmaceutical composition comprising the fusion protein of the present invention to an eye or ocular tissue, for example, topically administering the pharmaceutical composition to an eye, or administrating the pharmaceutical composition by injecting an eye (intraocular injection) or tissue associated with the eye, for example, administrating the liquid pharmaceutical composition by intraocular injection, periocular injection, subretinal injection, intravitreal injection, trans-septal injection, sub-scleral injection, intrachoroidal injection, intracameral injection, subconjunctival injection, subtenon injection, retrobulbar injection, peribulbar injection, or posterior juxtascleral delivery. The composition may also be administered, for example, to vitreous, optic nerve, aqueous humor, sclera, conjunctiva, the area between the sclera and conjunctiva, retinochoroidal tissue, macula, or other areas in or near the eye of the individual. Preferably, the composition is injected intravenously.

In some embodiments, the fusion protein of the present invention or a pharmaceutical composition thereof may be administered alone or in combination with other drugs.

In some embodiments of the above methods, central subfield thickness of the individual 2, 3 or 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration, as compared to central subfield thickness of the individual before administration, is decreased by 25 µm or more, for example, by 25-350 µm, preferably by 50-300 µm, more preferably by 50-200 µm, for example, by 25 µm, 50 µm, 75 µm, 100 µm, 125 µm, 150 µm, 175 µm, 200 µm, 225 µm, 250 µm, 275 µm, 300 µm, 325 µm or 350 µm; or is decreased by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

In some embodiments of the above methods, area of the choroidal neovascularization (CNV) of the individual 2, 3 or 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration, as compared to area of the choroidal neovascularization (CNV) of the individual before administration, is reduced by 0.1 mm² or more, for example, by 0.1-20 mm², preferably by 0.2-5 mm², more preferably by 0.3-2 mm², for example, by 0.1 mm², 0.2 mm², 0.3 mm², 0.4 mm², 0.5 mm², 0.6 mm², 0.7 mm², 0.8 mm², 0.9 mm², 1 mm², 1.1 mm², 1.2 mm², 1.3 mm², 1.4 mm², 1.5 mm², 1.6 mm², 1.7 mm², 1.8 mm², 1.9 mm², 2 mm², 2.1 mm², 2.2 mm², 2.3 mm², 2.4 mm², 2.5 mm², 2.6 mm², 2.7 mm², 2.8 mm², 2.9 mm², 3 mm², 3.1 mm², 3.2 mm², 3.3 mm², 3.4 mm², 3.5 mm², 3.6 mm², 3.7 mm², 3.8 mm², 3.9 mm², 4 mm², 4.1 mm², 4.2 mm², 4.3 mm², 4.4 mm², 4.5 mm², 4.6 mm², 4.7 mm², 4.8 mm², 4.9 mm², 5 mm², 5.5 mm², 6 mm ², 6.5 mm², 7 mm², 7.5 mm², 8 mm², 8.5 mm², 9 mm², 9.5 mm², 10 mm², 10.5 mm², 11 mm², 11.5 mm², 12 mm², 12.5 mm², 13 mm², 13.5 mm², 14 mm², 14.5 mm², 15 mm², 15.5 mm², 16 mm², 16.5 mm², 17 mm², 17.5 mm², 18 mm², 18.5 mm², 19 mm², 19.5 mm² or 20 mm²; or is reduced by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

In some embodiments of the above methods, best corrected visual acuity (BCVA) of the individual 2, 3 or 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration, as compared to best corrected visual acuity of the individual before administration, is improved by at least 1 ETDRS letter, preferably by at least 5 ETDRS letters, more preferably by 5-35 ETDRS letters, for example, by 5-25 ETDRS letters, for example, by 5-10, 5-15, 5-20, 10-15, 10-20, 10-25, 15-20 or 15-25 letters, for example, by 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 letters, or is improved by 5% or more, preferably by 10% or more, for example, by 10%-200%, preferably by 10%-150%, more preferably by 10%-100%, more preferably by 20%-100%, more preferably by 50%-100%, 60%-100%, 20%-80% or 30%-70%, more preferably by 50%-70%, more preferably by 60%-70%, for example, by 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190% or 200%.

In some embodiments of the above methods, the individual does not experience severe adverse events after administration.

In some embodiments of the above methods, the individual has an incidence of adverse events comparable to an individual receiving placebo after administration.

In some embodiments of the above methods, the individual is a human.

In some embodiments of the above methods, the method comprises intravitreally or intravenously administering to the individual the fusion protein at a single dose of about 0.01-10 mg/eye, preferably 0.05-8 mg/eye, more preferably 0.05-6 mg/eye, more preferably 0.5-5 mg/eye, more preferably 1-5 mg/eye, more preferably 2-5 mg/eye, for example, 0.5-2 mg/eye, 1-3 mg/eye, 2-4 mg/eye, 2-3 mg/eye or 3-5 mg/eye, for example, 0.05 mg/eye, 0.1 mg/eye, 0.15 mg/eye, 0.2 mg/eye, 0.25 mg/eye, 0.3 mg/eye, 0.35 mg/eye, 0.4 mg/eye, 0.45 mg/eye, 0.5 mg/eye, 0.55 mg/eye, 0.6 mg/eye, 0.65 mg/eye, 0.7 mg/eye, 0.75 mg/eye, 0.8 mg/eye, 0.85 mg/eye, 0.9 mg/eye, 0.95 mg/eye, 1 mg/eye, 1.1 mg/eye, 1.2 mg/eye, 1.3 mg/eye, 1.4 mg/eye, 1.5 mg/eye, 1.6 mg/eye, 1.7 mg/eye, 1.8 mg/eye, 1.9 mg/eye, 2 mg/eye, 2.1 mg/eye, 2.2 mg/eye, 2.3 mg/eye, 2.4 mg/eye, 2.5 mg/eye, 2.6 mg/eye, 2.7 mg/eye, 2.8 mg/eye, 2.9 mg/eye, 3 mg/eye, 3.1 mg/eye, 3.2 mg/eye, 3.3 mg/eye, 3.4 mg/eye, 3.5 mg/eye, 3.6 mg/eye, 3.7 mg/eye, 3.8 mg/eye, 3.9 mg/eye, 4 mg/eye, 4.1 mg/eye, 4.2 mg/eye, 4.3 mg/eye, 4.4 mg/eye, 4.5 mg/eye, 4.6 mg/eye, 4.7 mg/eye, 4.8 mg/eye, 4.9 mg/eye, 5 mg/eye, 5.1 mg/eye, 5.2 mg/eye, 5.3 mg/eye, 5.4 mg/eye, 5.5 mg/eye, 5.6 mg/eye, 5.7 mg/eye, 5.8 mg/eye, 5.9 mg/eye, 6 mg/eye, 6.1 mg/eye, 6.2 mg/eye, 6.3 mg/eye, 6.4 mg/eye, 6.5 mg/eye, 6.6 mg/eye, 6.7 mg/eye, 6.8 mg/eye, 6.9 mg/eye, 7 mg/eye, 7.1 mg/eye, 7.2 mg/eye, 7.3 mg/eye, 7.4 mg/eye, 7.5 mg/eye, 7.6 mg/eye, 7.7 mg/eye, 7.8 mg/eye, 7.9 mg/eye, 8 mg/eye, 8.5 mg/eye, 9 mg/eye, 9.5 mg/eye, 10 mg/eye, preferably 2 mg/eye or 4 mg/eye once a day, once every two days, twice a week, once a week, once every two weeks, once every four weeks, once every five weeks or once every six weeks, wherein central subfield thickness of the individual six or eight weeks after administration, as compared to central subfield thickness of the individual before administration, is decreased by 25 µm or more, for example, by 25-350 µm, preferably by 50-300 µm, more preferably by 50-200 µm, for example, by 25 µm, 50 µm, 75 µm, 100 µm, 125 µm, 150 µm, 175 µm, 200 µm, 225 µm, 250 µm, 275 µm, 300 µm, 325 µm or 350 µm; or is reduced by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

In some embodiments of the above methods, the method comprises intravitreally or intravenously administering to the individual the fusion protein at a single dose of about 0.01-10 mg/eye, preferably 0.05-8 mg/eye, more preferably 0.05-6 mg/eye, more preferably 0.5-5 mg/eye, more preferably 1-5 mg/eye, more preferably 2-5 mg/eye, for example, 0.5-2 mg/eye, 1-3 mg/eye, 2-4 mg/eye, 2-3 mg/eye or 3-5 mg/eye, for example, 0.05 mg/eye, 0.1 mg/eye, 0.15 mg/eye, 0.2 mg/eye, 0.25 mg/eye, 0.3 mg/eye, 0.35 mg/eye, 0.4 mg/eye, 0.45 mg/eye, 0.5 mg/eye, 0.55 mg/eye, 0.6 mg/eye, 0.65 mg/eye, 0.7 mg/eye, 0.75 mg/eye, 0.8 mg/eye, 0.85 mg/eye, 0.9 mg/eye, 0.95 mg/eye, 1 mg/eye, 1.1 mg/eye, 1.2 mg/eye, 1.3 mg/eye, 1.4 mg/eye, 1.5 mg/eye, 1.6 mg/eye, 1.7 mg/eye, 1.8 mg/eye, 1.9 mg/eye, 2 mg/eye, 2.1 mg/eye, 2.2 mg/eye, 2.3 mg/eye, 2.4 mg/eye, 2.5 mg/eye, 2.6 mg/eye, 2.7 mg/eye, 2.8 mg/eye, 2.9 mg/eye, 3 mg/eye, 3.1 mg/eye, 3.2 mg/eye, 3.3 mg/eye, 3.4 mg/eye, 3.5 mg/eye, 3.6 mg/eye, 3.7 mg/eye, 3.8 mg/eye, 3.9 mg/eye, 4 mg/eye, 4.1 mg/eye, 4.2 mg/eye, 4.3 mg/eye, 4.4 mg/eye, 4.5 mg/eye, 4.6 mg/eye, 4.7 mg/eye, 4.8 mg/eye, 4.9 mg/eye, 5 mg/eye, 5.1 mg/eye, 5.2 mg/eye, 5.3 mg/eye, 5.4 mg/eye, 5.5 mg/eye, 5.6 mg/eye, 5.7 mg/eye, 5.8 mg/eye, 5.9 mg/eye, 6 mg/eye, 6.1 mg/eye, 6.2 mg/eye, 6.3 mg/eye, 6.4 mg/eye, 6.5 mg/eye, 6.6 mg/eye, 6.7 mg/eye, 6.8 mg/eye, 6.9 mg/eye, 7 mg/eye, 7.1 mg/eye, 7.2 mg/eye, 7.3 mg/eye, 7.4 mg/eye, 7.5 mg/eye, 7.6 mg/eye, 7.7 mg/eye, 7.8 mg/eye, 7.9 mg/eye, 8 mg/eye, 8.5 mg/eye, 9 mg/eye, 9.5 mg/eye, 10 mg/eye, preferably 2 mg/eye or 4 mg/eye once a day, once every two days, twice a week, once a week, once every two weeks, once every four weeks, once every five weeks or once every six weeks, wherein area of the choroidal neovascularization (CNV) area of the individual six or eight weeks after administration, as compared to area of the choroidal neovascularization (CNV) of the individual before administration, is reduced by 0.1 mm² or more, for example, by 0.1-20 mm², preferably by 0.2-5 mm², more preferably by 0.3-2 mm², for example, by 0.1 mm², 0.2 mm², 0.3 mm², 0.4 mm², 0.5 mm², 0.6 mm², 0.7 mm², 0.8 mm², 0.9 mm², 1 mm², 1.1 mm², 1.2 mm², 1.3 mm², 1.4 mm², 1.5 mm², 1.6 mm², 1.7 mm², 1.8 mm², 1.9 mm², 2 mm², 2.1 mm², 2.2 mm², 2.3 mm², 2.4 mm², 2.5 mm², 2.6 mm², 2.7 mm², 2.8 mm², 2.9 mm², 3 mm², 3.1 mm², 3.2 mm², 3.3 mm², 3.4 mm², 3.5 mm², 3.6 mm², 3.7 mm², 3.8 mm², 3.9 mm², 4 mm², 4.1 mm², 4.2 mm², 4.3 mm², 4.4 mm², 4.5 mm², 4.6 mm², 4.7 mm², 4.8 mm², 4.9 mm², 5 mm², 5.5 mm², 6 mm², 6.5 mm², 7 mm², 7.5 mm², 8 mm², 8.5 mm², 9 mm², 9.5 mm², 10 mm², 10.5 mm², 11 mm², 11.5 mm², 12 mm², 12.5 mm², 13 mm², 13.5 mm², 14 mm², 14.5 mm², 15 mm², 15.5 mm², 16 mm², 16.5 mm², 17 mm², 17.5 mm², 18 mm², 18.5 mm², 19 mm², 19.5 mm² or 20 mm²; or is reduced by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

In some embodiments of the above methods, the method comprises intravitreally or intravenously administering to the individual the fusion protein at a single dose of about 0.01-10 mg/eye, preferably 0.05-8 mg/eye, more preferably 0.05-6 mg/eye, more preferably 0.5-5 mg/eye, more preferably 1-5 mg/eye, more preferably 2-5 mg/eye, for example, 0.5-2 mg/eye, 1-3 mg/eye, 2-4 mg/eye, 2-3 mg/eye or 3-5 mg/eye, for example, 0.05 mg/eye, 0.1 mg/eye, 0.15 mg/eye, 0.2 mg/eye, 0.25 mg/eye, 0.3 mg/eye, 0.35 mg/eye, 0.4 mg/eye, 0.45 mg/eye, 0.5 mg/eye, 0.55 mg/eye, 0.6 mg/eye, 0.65 mg/eye, 0.7 mg/eye, 0.75 mg/eye, 0.8 mg/eye, 0.85 mg/eye, 0.9 mg/eye, 0.95 mg/eye, 1 mg/eye, 1.1 mg/eye, 1.2 mg/eye, 1.3 mg/eye, 1.4 mg/eye, 1.5 mg/eye, 1.6 mg/eye, 1.7 mg/eye, 1.8 mg/eye, 1.9 mg/eye, 2 mg/eye, 2.1 mg/eye, 2.2 mg/eye, 2.3 mg/eye, 2.4 mg/eye, 2.5 mg/eye, 2.6 mg/eye, 2.7 mg/eye, 2.8 mg/eye, 2.9 mg/eye, 3 mg/eye, 3.1 mg/eye, 3.2 mg/eye, 3.3 mg/eye, 3.4 mg/eye, 3.5 mg/eye, 3.6 mg/eye, 3.7 mg/eye, 3.8 mg/eye, 3.9 mg/eye, 4 mg/eye, 4.1 mg/eye, 4.2 mg/eye, 4.3 mg/eye, 4.4 mg/eye, 4.5 mg/eye, 4.6 mg/eye, 4.7 mg/eye, 4.8 mg/eye, 4.9 mg/eye, 5 mg/eye, 5.1 mg/eye, 5.2 mg/eye, 5.3 mg/eye, 5.4 mg/eye, 5.5 mg/eye, 5.6 mg/eye, 5.7 mg/eye, 5.8 mg/eye, 5.9 mg/eye, 6 mg/eye, 6.1 mg/eye, 6.2 mg/eye, 6.3 mg/eye, 6.4 mg/eye, 6.5 mg/eye, 6.6 mg/eye, 6.7 mg/eye, 6.8 mg/eye, 6.9 mg/eye, 7 mg/eye, 7.1 mg/eye, 7.2 mg/eye, 7.3 mg/eye, 7.4 mg/eye, 7.5 mg/eye, 7.6 mg/eye, 7.7 mg/eye, 7.8 mg/eye, 7.9 mg/eye, 8 mg/eye, 8.5 mg/eye, 9 mg/eye, 9.5 mg/eye, 10 mg/eye, preferably 2 mg/eye or 4 mg/eye once a day, once every two days, twice a week, once a week, once every two weeks, once every four weeks, once every five weeks or once every six weeks, wherein best corrected visual acuity (BCVA) of the individual six or eight weeks after administration, as compared to best corrected visual acuity (BCVA) of the individual before administration, is improved by at least 1 ETDRS letter, preferably by at least 5 ETDRS letters, more preferably by 5-35 ETDRS letters, for example, by 5-25 ETDRS letters, for example, by 5-10, 5-15, 5-20, 10-15, 10-20, 10-25, 15-20 or 15-25 letters, for example, by 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 letters; or is improved by 5% or more, preferably by 10% or more, for example, by 10%-200%, preferably by 10%-150%, more preferably by 10%-100%, more preferably by 20%-100%, more preferably by 50%-100%, 60%-100%, 20%-80% or 30%-70%, more preferably by 50%-70%, more preferably by 60%-70%, for example, by 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190% or 200%.

### II. Use

In a second aspect, the present invention relates to use of the fusion protein inhibiting a VEGF pathway and a complement pathway in the manufacture of a medicament for preventing or treating age-related macular degeneration (AMD) in an individual.

In a third aspect, the present invention relates to a fusion protein inhibiting a VEGF pathway and a complement pathway for use in preventing or treating age-related macular degeneration (AMD) in an individual.

The second and third aspects are further defined as follows:

In an embodiment, the fusion protein comprises a sequence of SEQ ID NO: 1:

DTGRPFVEMYSEIPEIIHMTEGRELVIPCRVTSPNITVTLKKFPLDTLIPDGKKIIWDSRKGFIISNATYKEIG
LLTCEATVNGHLYKTNYLTHRQTNTIIDVVLSPSHGIELSVGEKLVLNCTARTELNVGIDFNWEYPSSKH
QHKKLVNRDLKTQSGSEMKKFLSTLTIDGVTRSDQGLYTCAASSGLMTKKNSTFVRVHEKDKTHTCPPC
PAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHY
TQKSLSLSPGKGGGGGGQCNAPEWLPFARPTNLTDEFEFPIGTYLKYECRPGYYGRPFSIICLKNSVWTGA
KDRCRRKSCRNPPDPVNGMVHVIKDIQFGSQIKYSCTKGYRLIGSSSATCIISGNTVIWDNETPICDRIPCG
LPPTITNGDFISTNRENFHYGSVVTYRCNPGSGGRKVFELVGEPSIYCTSNDDQVGIWSGPAPQCI (SEQ ID
NO: 1).

In a preferred embodiment, the sequence of the fusion protein is a sequence set forth in SEQ ID NO: 1. For convenience of expression, the fusion protein is referred to herein as IBI302 and the preparation method thereof is described in WO2013082563A1.

In an embodiment, the AMD is wet AMD.

In an embodiment, the wet AMD has one or more of the following symptoms and signs: visual acuity decrease, metamorphopsia, central scotoma, dyslexia, macular retinal swelling, fundus hemorrhage, neovascularization, scar fibrosis, geographic atrophy, or any combination thereof.

In an embodiment, the wet AMD has choroidal neovascularization (CNV).

In an embodiment, the wet AMD is AMD having the following characteristics:
1) active CNV under the macular fovea secondary to wet AMD occurs in a study eye; and/or
2) central subfield thickness is ≥ 250 µm through optical coherence tomography (OCT) scanning.

In an embodiment, the individual is a human individual.

In a preferred embodiment, the individual is a human individual having the disease, symptoms, and/or characteristics as described in any of the above embodiments.

In a preferred embodiment, the individual is a human individual who is a wet AMD patient accompanied by geographic atrophy.

In a preferred embodiment, the individual suffers from wet AMD with the following characteristics:
1) active CNV under the macular fovea secondary to wet AMD occurs in a study eye; and/or
2) central subfield thickness is ≥ 250 µm through optical coherence tomography (OCT) scanning.

In a preferred embodiment, the treatment may achieve one or more of the following effects in the individual:
1) central subfield thickness is decreased from a baseline;
2) area of the choroidal neovascularization (CNV) is reduced;
3) best corrected visual acuity (BCVA) is improved from a baseline;
4) onset of the geographic atrophy in the wet AMD patient is lagged; and/or
5) onset of the geographic atrophy in the wet AMD patient is slowed.

In a preferred embodiment, the treatment may achieve one or more of the following effects in the individual:
1) best corrected visual acuity (BCVA) is improved;
2) central subfield thickness is decreased; and/or
3) choroidal neovascularization (CNV) area is reduced.

In a preferred embodiment, the treatment may achieve one or more of the following effects in the individual:
1) best corrected visual acuity (BCVA), as compared to a baseline, is improved by at least 1 ETDRS letter, preferably by at least 5 ETDRS letters, more preferably by 5-35 ETDRS letters, for example, by 5-25 ETDRS letters, for example, by 5-10, 5-15, 5-20, 10-15, 10-20, 10-25, 15-20 or 15-25 letters, for example, by 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 letters; and/or
2) best corrected visual acuity (BCVA), as compared to a baseline, is improved by 5% or more, preferably by 10% or more, for example, by 10%-200%, preferably by 10%-150%, more preferably by 10%-100%, more preferably by 20%-100%, more preferably by 50%-100%, 60%-100%, 20%-80% or 30%-70%, more preferably by 50%-70%, more preferably by 60%-70%, for example, by 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190% or 200%, and/or
3) central subfield thickness (CST), as compared to a baseline, is decreased by 25 µm or more, for example, by 25-350 µm, preferably by 50-300 µm, more preferably by 50-200 µm, for example, by 25 µm, 50 µm, 75 µm, 100 µm, 125 µm, 150 µm, 175 µm, 200 µm, 225 µm, 250 µm, 275 µm, 300 µm, 325 µm or 350 µm; or is decreased by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%; and/or
4) area of the choroidal neovascularization (CNV), as compared to a baseline, is reduced by 0.1 mm² or more, for example, by 0.1-20 mm², preferably by 0.2-5 mm², more preferably by 0.3-2 mm², for example, by 0.1 mm², 0.2 mm², 0.3 mm², 0.4 mm², 0.5 mm², 0.6 mm², 0.7 mm², 0.8 mm², 0.9 mm², 1 mm², 1.1 mm², 1.2 mm², 1.3 mm², 1.4 mm², 1.5 mm², 1.6 mm², 1.7 mm², 1.8 mm², 1.9 mm², 2 mm², 2.1 mm², 2.2 mm², 2.3 mm², 2.4 mm², 2.5 mm², 2.6 mm², 2.7 mm², 2.8 mm², 2.9 mm², 3 mm², 3.1 mm², 3.2 mm², 3.3 mm², 3.4 mm², 3.5 mm², 3.6 mm², 3.7 mm², 3.8 mm², 3.9 mm², 4 mm², 4.1 mm², 4.2 mm², 4.3 mm², 4.4 mm², 4.5 mm², 4.6 mm², 4.7 mm², 4.8 mm², 4.9 mm², 5 mm², 5.5 mm², 6 mm², 6.5 mm², 7 mm², 7.5 mm², 8 mm², 8.5 mm², 9 mm², 9.5 mm², 10 mm², 10.5 mm², 11 mm², 11.5 mm², 12 mm², 12.5 mm², 13 mm², 13.5 mm², 14 mm², 14.5 mm², 15 mm², 15.5 mm², 16 mm², 16.5 mm², 17 mm², 17.5 mm², 18 mm², 18.5 mm², 19 mm², 19.5 mm² or 20 mm²; or is reduced by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

In some embodiments, the fusion protein of the present invention is administered at a frequency of three times a day, twice a day, once a day, once every two days, twice a week, once a week, once every two weeks, once every four weeks, once every five weeks, once every six weeks, once every seven weeks, once every eight weeks, once a month, once every two months, or longer. The fusion protein of the present invention may be administered in a cycle of one week, two weeks, three weeks, one month, two months, three months or longer, and the intervals between every two administration cycles may be the same or different. In a preferred embodiment, the fusion protein of the present invention is administered once or twice, preferably once, in each administration cycle, in the mode of administration of administering on the first day of each cycle.

In each of the above embodiments, a single dose of the fusion protein of the present invention is selected from 0.01-10 mg/eye, preferably 0.05-8 mg/eye, more preferably 0.05-6 mg/eye, more preferably 0.5-5 mg/eye, more preferably 1-5 mg/eye, more preferably 2-5 mg/eye, for example, 0.5-2 mg/eye, 1-3 mg/eye, 2-4 mg/eye, 2-3 mg/eye or 3-5 mg/eye, for example, 0.05 mg/eye, 0.1 mg/eye, 0.15 mg/eye, 0.2 mg/eye, 0.25 mg/eye, 0.3 mg/eye, 0.35 mg/eye, 0.4 mg/eye, 0.45 mg/eye, 0.5 mg/eye, 0.55 mg/eye, 0.6 mg/eye, 0.65 mg/eye, 0.7 mg/eye, 0.75 mg/eye, 0.8 mg/eye, 0.85 mg/eye, 0.9 mg/eye, 0.95 mg/eye, 1 mg/eye, 1.1 mg/eye, 1.2 mg/eye, 1.3 mg/eye, 1.4 mg/eye, 1.5 mg/eye, 1.6 mg/eye, 1.7 mg/eye, 1.8 mg/eye, 1.9 mg/eye, 2 mg/eye, 2.1 mg/eye, 2.2 mg/eye, 2.3 mg/eye, 2.4 mg/eye, 2.5 mg/eye, 2.6 mg/eye, 2.7 mg/eye, 2.8 mg/eye, 2.9 mg/eye, 3 mg/eye, 3.1 mg/eye, 3.2 mg/eye, 3.3 mg/eye, 3.4 mg/eye, 3.5 mg/eye, 3.6 mg/eye, 3.7 mg/eye, 3.8 mg/eye, 3.9 mg/eye, 4 mg/eye, 4.1 mg/eye, 4.2 mg/eye, 4.3 mg/eye, 4.4 mg/eye, 4.5 mg/eye, 4.6 mg/eye, 4.7 mg/eye, 4.8 mg/eye, 4.9 mg/eye, 5 mg/eye, 5.1 mg/eye, 5.2 mg/eye, 5.3 mg/eye, 5.4 mg/eye, 5.5 mg/eye, 5.6 mg/eye, 5.7 mg/eye, 5.8 mg/eye, 5.9 mg/eye, 6 mg/eye, 6.1 mg/eye, 6.2 mg/eye, 6.3 mg/eye, 6.4 mg/eye, 6.5 mg/eye, 6.6 mg/eye, 6.7 mg/eye, 6.8 mg/eye, 6.9 mg/eye, 7 mg/eye, 7.1 mg/eye, 7.2 mg/eye, 7.3 mg/eye, 7.4 mg/eye, 7.5 mg/eye, 7.6 mg/eye, 7.7 mg/eye, 7.8 mg/eye, 7.9 mg/eye, 8 mg/eye, 8.5 mg/eye, 9 mg/eye, 9.5 mg/eye or 10 mg/eye, preferably 2 mg/eye or 4 mg/eye.

In some embodiments, the fusion protein of the present invention or a pharmaceutical composition thereof may be administered alone or in combination with other drugs.

In some embodiments, central subfield thickness of the individual 2, 3 or 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration, as compared to central subfield thickness of the individual before administration, is decreased by 25 µm or more, for example, by 25-350 µm, preferably by 50-300 µm, more preferably by 50-200 µm, for example, by 25 µm, 50 µm, 75 µm, 100 µm, 125 µm, 150 µm, 175 µm, 200 µm, 225 µm, 250 µm, 275 µm, 300 µm, 325 µm or 350 µm; or is decreased by 5% or more, for example, by 10%-70%, preferably by 10%-60%, more preferably by 30%-50%, more preferably by 40%-50%, for example, 5%, 10%, 15%, 20%, 25%, 30%, 35%, 40%, 45%, 50%, 55%, 60%, 65% or 70%.

In some embodiments, area of the choroidal neovascularization (CNV) of the individual 2, 3 or 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration, as compared to area of the choroidal neovascularization (CNV) of the individual before administration, is reduced by 0.1 mm² or more, for example, by 0.1-20 mm², preferably by 0.2-5 mm², more preferably by 0.3-2 mm², for example, by 0.1 mm², 0.2 mm², 0.3 mm², 0.4 mm², 0.5 mm², 0.6 mm², 0.7 mm², 0.8 mm², 0.9 mm², 1 mm², 1.1 mm², 1.2 mm², 1.3 mm², 1.4 mm², 1.5 mm², 1.6 mm², 1.7 mm², 1.8 mm², 1.9 mm², 2 mm², 2.1 mm², 2.2 mm², 2.3 mm², 2.4 mm², 2.5 mm², 2.6 mm², 2.7 mm², 2.8 mm², 2.9 mm², 3 mm², 3.1 mm², 3.2 mm², 3.3 mm², 3.4 mm², 3.5 mm², 3.6 mm², 3.7 mm², 3.8 mm², 3.9 mm², 4 mm², 4.1 mm², 4.2 mm², 4.3 mm², 4.4 mm², 4.5 mm², 4.6 mm², 4.7 mm², 4.8 mm², 4.9 mm², 5 mm², 5.5 mm², 6 mm², 6.5 mm², 7 mm², 7.5 mm², 8 mm², 8.5 mm², 9 mm², 9.5 mm², 10 mm², 10.5 mm², 11 mm², 11.5 mm², 12 mm², 12.5 mm², 13 mm², 13.5 mm², 14 mm², 14.5 mm², 15 mm², 15.5 mm², 16 mm², 16.5 mm², 17 mm², 17.5 mm², 18 mm², 18.5 mm², 19 mm², 19.5 mm² or 20 mm²; or is reduced by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

In some embodiments, best corrected visual acuity (BCVA) of the individual 2, 3 or 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration, as compared to best corrected visual acuity of the individual before administration, is improved by at least 1 ETDRS letter, preferably by at least 5 ETDRS letters, more preferably by 5-35 ETDRS letters, for example, by 5-25 ETDRS letters, for example, by 5-10, 5-15, 5-20, 10-15, 10-20, 10-25, 15-20 or 15-25 letters, for example, by 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 letters; or is improved by 5% or more, preferably by 10% or more, for example, by 10%-200%, preferably by 10%-150%, more preferably by 10%-100%, more preferably by 20%-100%, more preferably by 50%-100%, 60%-100%, 20%-80% or 30%-70%, more preferably by 50%-70%, more preferably by 60%-70%, for example, by 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190% or 200%.

In some embodiments, the individual does not experience severe adverse events after administration.

In some embodiments, the individual has an incidence of adverse events comparable to an individual receiving placebo after administration.

In some embodiments, the individual is a human.

In some embodiments, the fusion protein is administered to the individual in accordance with the following scheme: intravitreally or intravenously administering the fusion protein at a single dose of about 0.01-10 mg/eye, preferably 0.05-8 mg/eye, more preferably 0.05-6 mg/eye, more preferably 0.5-5 mg/eye, more preferably 1-5 mg/eye, more preferably 2-5 mg/eye, for example, 0.5-2 mg/eye, 1-3 mg/eye, 2-4 mg/eye, 2-3 mg/eye or 3-5 mg/eye once a day, once every two days, twice a week, once a week, once every two weeks, once every four weeks, once every five weeks or once every six weeks, wherein central subfield thickness of the individual six or eight weeks after administration, as compared to central subfield thickness of the individual before administration, is decreased by 25 µm or more, for example, by 25-350 µm, preferably by 50-300 µm, more preferably by 50-200 µm, for example, by 25 µm, 50 µm, 75 µm, 100 µm, 125 µm, 150 µm, 175 µm, 200 µm, 225 µm, 250 µm, 275 µm, 300 µm, 325 µm or 350 µm; or is decreased by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

In some embodiments, the fusion protein is administered to the individual in accordance with the following scheme: intravitreally or intravenously administering the fusion protein at a single dose of about 0.01-10 mg/eye, preferably 0.05-8 mg/eye, more preferably 0.05-6 mg/eye, more preferably 0.5-5 mg/eye, more preferably 1-5 mg/eye, more preferably 2-5 mg/eye, for example, 0.5-2 mg/eye, 1-3 mg/eye, 2-4 mg/eye, 2-3 mg/eye or 3-5 mg/eye once a day, once every two days, twice a week, once a week, once every two weeks, once every four weeks, once every five weeks or once every six weeks, wherein area of the choroidal neovascularization (CNV) of the individual six or eight weeks after administration, as compared to area of the choroidal neovascularization (CNV) of the individual before administration, is reduced by more than 0.1 mm², for example, by 0.1-20 mm², preferably by 0.2-5 mm², more preferably by 0.3-2 mm², for example, by 0.1 mm², 0.2 mm², 0.3 mm², 0.4 mm², 0.5 mm², 0.6 mm², 0.7 mm², 0.8 mm², 0.9 mm², 1 mm², 1.1 mm², 1.2 mm², 1.3 mm², 1.4 mm², 1.5 mm², 1.6 mm², 1.7 mm², 1.8 mm², 1.9 mm², 2 mm², 2.1 mm², 2.2 mm², 2.3 mm², 2.4 mm², 2.5 mm², 2.6 mm², 2.7 mm², 2.8 mm², 2.9 mm², 3 mm², 3.1 mm², 3.2 mm², 3.3 mm², 3.4 mm², 3.5 mm², 3.6 mm², 3.7 mm², 3.8 mm², 3.9 mm², 4 mm², 4.1 mm², 4.2 mm², 4.3 mm², 4.4 mm², 4.5 mm², 4.6 mm², 4.7 mm², 4.8 mm², 4.9 mm², 5 mm², 5.5 mm², 6 mm², 6.5 mm², 7 mm², 7.5 mm², 8 mm², 8.5 mm², 9 mm², 9.5 mm², 10 mm², 10.5 mm², 11 mm², 11.5 mm², 12 mm², 12.5 mm², 13 mm², 13.5 mm², 14 mm², 14.5 mm², 15 mm², 15.5 mm², 16 mm², 16.5 mm², 17 mm², 17.5 mm², 18 mm², 18.5 mm², 19 mm², 19.5 mm² or 20 mm²; or is reduced by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

In some embodiments, the fusion protein is administered to the individual in accordance with the following scheme: intravitreally or intravenously administering the fusion protein at a single dose of about 0.01-10 mg/eye, preferably 0.05-8 mg/eye, more preferably 0.05-6 mg/eye, more preferably 0.5-5 mg/eye, more preferably 1-5 mg/eye, more preferably 2-5 mg/eye, for example, 0.5-2 mg/eye, 1-3 mg/eye, 2-4 mg/eye, 2-3 mg/eye or 3-5 mg/eye once a day, once every two days, twice a week, once a week, once every two weeks, once every four weeks, once every five weeks or once every six weeks, wherein best corrected visual acuity (BCVA) of the individual six or eight weeks after administration, as compared to best corrected visual acuity (BCVA) of the individual before administration, is improved by at least 1 ETDRS letter, preferably by at least 5 ETDRS letters, more preferably by 5-35 ETDRS letters, for example, by 5-25 ETDRS letters, for example, by 5-10, 5-15, 5-20, 10-15, 10-20, 10-25, 15-20 or 15-25 letters, for example, by 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 letters; or is improved by 5% or more, preferably by 10% or more, for example, by 10%-200%, preferably by 10%-150%, more preferably by 10%-100%, more preferably by 20%-100%, more preferably by 50%-100%, 60%-100%, 20%-80% or 30%-70%, more preferably by 50%-70%, more preferably by 60%-70%, for example, by 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190% or 200%.

It should be understood that technical schemes obtained from any combination of any of the technical features described in the second to third aspects above and any of the technical features of the technical scheme described in the first aspect are also included in the present application.

### III. Single Pharmaceutical Dosage Unit or Pharmaceutical Kit

In a fourth aspect, the present invention relates to a single pharmaceutical dosage unit, which comprises the fusion protein inhibiting a VEGF pathway and a complement pathway.

In an embodiment, the fusion protein comprises a sequence of SEQ ID NO: 1:

DTGRPFVEMYSEIPEIIHMTEGRELVIPCRVTSPNITVTLKKFPLDTLIPDGKKIIWDSRKGFIISNATYKEIG
LLTCEATVNGHLYKTNYLTHRQTNTIIDVVLSPSHGIELSVGEKLVLNCTARTELNVGIDFNWEYPSSKH
QHKKLVNRDLKTQSGSEMKKFLSTLTIDGVTRSDQGLYTCAASSGLMTKKNSTFVRVHEKDKTHTCPPC
PAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHY
TQKSLSLSPGKGGGGGGQCNAPEWLPFARPTNLTDEFEFPIGTYLKYECRPGYYGRPFSIICLKNSVWTGA
KDRCRRKSCRNPPDPVNGMVHVIKDIQFGSQIKYSCTKGYRLIGSSSATCIISGNTVIWDNETPICDRIPCG
LPPTITNGDFISTNRENFHYGSVVTYRCNPGSGGRKVFELVGEPSIYCTSNDDQVGIWSGPAPQCI (SEQ ID
NO: 1).

In a preferred embodiment, the sequence of the fusion protein is a sequence set forth in SEQ ID NO: 1. For convenience of expression, the fusion protein is referred to herein as IBI302 and the preparation method thereof is described in WO2013082563A1.

In some embodiments, the single pharmaceutical dosage unit comprises the fusion protein at the following doses: 0.01-10 mg, preferably 0.05-8 mg, more preferably 0.05-6 mg, more preferably 0.5-5 mg, more preferably 1-5 mg, more preferably 2-5 mg, for example, 0.5-2 mg, 1-3 mg, 2-4 mg, 2-3 mg or 3-5 mg, for example, 0.05 mg, 0.1 mg, 0.15 mg, 0.2 mg, 0.25 mg, 0.3 mg, 0.35 mg, 0.4 mg, 0.45 mg, 0.5 mg, 0.55 mg, 0.6 mg, 0.65 mg, 0.7 mg, 0.75 mg, 0.8 mg, 0.85 mg, 0.9 mg, 0.95 mg, 1 mg, 1.1 mg, 1.2 mg, 1.3 mg, 1.4 mg, 1.5 mg, 1.6 mg, 1.7 mg, 1.8 mg, 1.9 mg, 2 mg, 2.1 mg, 2.2 mg, 2.3 mg, 2.4 mg, 2.5 mg, 2.6 mg, 2.7 mg, 2.8 mg, 2.9 mg, 3 mg, 3.1 mg, 3.2 mg, 3.3 mg, 3.4 mg, 3.5 mg, 3.6 mg, 3.7 mg, 3.8 mg, 3.9 mg, 4 mg, 4.1 mg, 4.2 mg, 4.3 mg, 4.4 mg, 4.5 mg, 4.6 mg, 4.7 mg, 4.8 mg, 4.9 mg, 5 mg, 5.1 mg, 5.2 mg, 5.3 mg, 5.4 mg, 5.5 mg, 5.6 mg, 5.7 mg, 5.8 mg, 5.9 mg, 6 mg, 6.1 mg, 6.2 mg, 6.3 mg, 6.4 mg, 6.5 mg, 6.6 mg, 6.7 mg, 6.8 mg, 6.9 mg, 7 mg, 7.1 mg, 7.2 mg, 7.3 mg, 7.4 mg, 7.5 mg, 7.6 mg, 7.7 mg, 7.8 mg, 7.9 mg, 8 mg, 8.5 mg, 9 mg, 9.5 mg or 10 mg, preferably 2 mg or 4 mg.

In a fifth aspect, the present invention relates to a pharmaceutical kit, which comprises the fusion protein according to any of the above embodiments of the single pharmaceutical dosage unit.

In a sixth aspect, the present invention relates to use of the above single pharmaceutical dosage unit or the above pharmaceutical kit in the manufacture of a medicament for preventing or treating age-related macular degeneration (AMD).

In a seventh aspect, the present invention relates to the above single pharmaceutical dosage unit or the above pharmaceutical kit for use in the prevention or treatment of age-related macular degeneration (AMD).

It should be understood that technical schemes obtained from any combination of any of the technical features described in the fourth to seventh aspects above and any of the technical features of the technical scheme described in the first to third aspects are also included in the present application.

### Definitions

The terms used herein have the following definitions. If the term is not defined herein, its definition may be referred to the patent application WO2013082563A1. If no definitions are given in both patent applications, the terms used in the present invention have the meanings commonly understood in the art.

For the purpose of explaining this specification, the following definitions will be used, and wherever appropriate, terms used in the singular form may also include the plural form, and vice versa. It should be understood that the terms used herein are for the purpose of describing specific embodiments only, and are not intended to be limiting. As used herein, the term "and/or" refers to any one of the options or two or more of the options.

As used herein, the term "comprise" or "include" means that the described elements, integers or steps are included, but not to the exclusion of any other elements, integers or steps. The term "comprise" or "include" used herein, unless otherwise specified, also encompasses the situation where the entirety consists of the described elements, integers or steps. For example, when referring to an antibody variable region "comprising" a particular sequence, it is also intended to encompass an antibody variable region consisting of the particular sequence.

The "individual" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the individual is a human, including a child, a teenager or an adult.

The term "administration in combination" refers to administering two or more therapeutic agents to treat a disease as described herein. Such administration includes co-administration of the therapeutic agents in a substantially simultaneous manner, for example, in a single composition with a fixed proportion of active ingredients. Alternatively, such administration includes co-administration of the active ingredients in a variety of or separate containers (such as tablets, capsules, powder and liquid). The powder and/or liquid can be reconstituted or diluted to a desired dosage before administration. In addition, such administration also includes using each type of the therapeutic agents at approximately the same time or in a sequential manner at different times. In any case, the therapeutic regimen will provide the beneficial effect of the pharmaceutical combination in the treatment of disorders or symptoms described herein.

As used herein, "treatment" (or "treat" or "treating") refers to slowing, interrupting, arresting, alleviating, stopping, lowering, or reversing the progression or severity of an existing symptom, disorder, condition, or disease.

As used herein, "prevention" (or "prevent" or "preventing") includes the inhibition of the onset or progression of symptoms of a disease or disorder, or a specific disease or disorder. In some embodiments, individuals with family medical history are candidates for a prophylactic regimen. Generally, the term "prevention" (or "prevent" or "preventing") refers to the administration of a drug prior to the onset of disorders or symptoms, particularly in individuals at risk.

The term "effective amount" refers to an amount or dose of the formulation or fusion protein of the present invention that produces a desired effect in a treated patient after the administration to the patient in a single dose or multiple doses. The effective amount can be easily determined by an attending physician as a person skilled in the art by considering a variety of factors as follows: species such as mammals; its size, age, and general health condition; the specific disease involved; the extent or severity of the disease; response in an individual patient; specific antibody administered; mode of administration; bioavailability characteristics of the administered formulation; selected administration regimen; and use of any concomitant therapy.

The "therapeutically effective amount" refers to an amount effective to achieve a desired therapeutic result at a necessary dosage for a necessary period of time. The therapeutically effective amount of the formulation, antibody or antibody fragment described herein, or conjugate or composition thereof can vary depending on a variety of factors such as disease state, age, sex and weight of an individual, and the ability of the antibody or antibody portion to elicit a desired response in the individual. The therapeutically effective amount is also such an amount in which any toxic or undesired effect of the formulation, antibody or antibody fragment or conjugate or composition thereof is inferior to the therapeutically beneficial effect.

The "prophylactically effective amount" refers to an amount effective to achieve a desired prophylactic result at a necessary dosage for a necessary period of time. Generally, since a prophylactic dose is administered in a subject before or at an earlier stage of a disease, a prophylactically effective amount will be less than a therapeutically effective amount.

The term "fusion protein" refers to a polypeptide having two or more moieties covalently linked together, wherein each of the moieties is derived from a different protein. The two or more moieties may be linked directly by a single peptide bond or by a peptide linker comprising one or more amino acid residues. Typically, the two moieties and the linker will be in a reading frame with each other and produced using recombinant techniques. The term "fusion protein inhibiting the VEGF pathway and the complement pathway" comprises a complement inhibitory domain (CID), a VEGF inhibitory domain (VID) and a half-life extending domain. In a preferred embodiment of the present invention, the VEGF fusion protein is selected from SEQ ID NO: 40 in WO2013082563A1, and the "CID", "VID" and "half-life extending domain" are referred to WO2013082563A1. The term "central retina", located in the center of the retina, is the most sensitive area of vision where cone cells responsible for photopic and color vision are distributed, so that any lesion involving this area will cause a significant decrease in central vision, and may also be combined with color vision abnormalities, metamorphopsia, central scotoma and the like, and the lesion may be manifested as hemorrhage, edema, increased thickness, atrophy and the like. In addition, the macula is located most centrally in the central retinal region.

The term "choroidal neovascularization" refers to a proliferating vessel from the choroidal capillaries that expands through the cleft of the Bruch's membrane, and is proliferated between the Bruch's membrane and the retinal pigment epithelium, or between the neural retina and the retinal pigment epithelium, or between the retinal pigment epithelium and the choroid, and many diseases involving the RPE-Bruch membrane-choroidal capillary complex can result in the choroidal neovascularization.

The term "best corrected visual acuity" refers to the vision measured after complete correction of the refractive error of the eye, and represents the actual visual ability of the eyeball.

As used herein, the term "formulation" or "pharmaceutical composition" refers to a composition which is suitably administered to animals, preferably mammals (including humans) and comprises at least one active ingredient and at least one non-active ingredient. The "liquid formulation" or "liquid composition" refers to a formulation in liquid form. The liquid composition of the present invention comprises (i) the fusion protein of the present invention; (ii) a buffer; and (iii) a vehicle. The composition of the formulation of the present invention may be shown in the above embodiments involving liquid pharmaceutical compositions. The liquid formulation of the present invention is preferably an injection.

The "pharmaceutically acceptable carrier" refers to an ingredient in a pharmaceutical formulation other than an active ingredient that is not toxic to the subject. The pharmaceutically acceptable carriers include, but are not limited to a buffer, an excipient, a stabilizer or a preservative.

As used herein, the "buffer" refers to a pH buffer. For example, the buffer is selected from histidine, glutamate, phosphate, acetate, citrate and tris(hydroxymethyl)aminomethane.

As used herein, the term "solvent" refers to a liquid that is used to dissolve or suspend active ingredients and non-active ingredients to form a liquid formulation. Solvents that can be used in the present invention include, but are not limited to water for injection, organic solvents for injection (including but not limited to oil for injection, ethanol, and propylene glycol), and a combination thereof.

As used herein, the term "single pharmaceutical dosage unit" refers to a single pharmaceutical dosage form comprising the fusion protein of the present invention to be administered to a patient at the time of administration, e.g., a vial, ampoule, pre-filled needle or pre-filled syringe for injection, containing a solution or lyophilized powder of the drug.

The term "about" used in combination with a numerical value is intended to encompass the numerical values in a range from a lower limit less than the specified numerical value by 10% to an upper limit greater than the specified numerical value by 10%.

### BRIEF DESCRIPTION OF THE DRAWING

FIG. 1 depicts the inhibitory effect of IBI302 on VEGF-A induced HUVEC proliferation.
FIG. 2 depicts the inhibitory effect of IBI302 on hemolysis mediated by the classical pathway.
FIG. 3 depicts the inhibitory effect of IBI302 on hemolysis mediated by the alternative pathway.
FIG. 4 depicts the inhibitory effect of IBI302 at various doses on CNV. ^{∗}P < 0.05 vs. PBS group; #P < 0.05 vs. IBI302 1 µg, 3 µg or 5 µg group.
FIG. 5 depicts the inhibition of IBI302 on the formation and leakage of CNV. ^{∗}P < 0.05 vs. PBS group; #P < 0.05 vs. IBI302 10 µg group.
FIG. 6 depicts the inhibitory effect of IBI302 on inflammatory cell aggregation. ^{∗}P < 0.05 vs. PBS group; #P < 0.05 vs. IBI302 10 µg group.
FIG. 7 depicts the effect of IBI302 on fundus fluorescence leakage from a rhesus CNV model (fluorescence angiography).
FIG. 8 depicts representative plots of immunohistochemical staining for CD31 in choroid.
FIG. 9 depicts representative plots of immunohistochemical staining for C5b-9 in choroid.
FIG. 10 depicts concentration-time curves in serum of rhesus monkeys after intravenous and intravitreal injections of IBI302.
FIG. 11 depicts concentration-time curves in aqueous humor and vitreous humor of rhesus monkeys after intravitreal injection of IBI302.
FIG. 12 depicts concentration-time curves in crystalline lens, cornea, iris, retina, choroid, optic nerve and sclera of rhesus monkeys after intravitreal injection of IBI302.
FIG. 13 depicts mean plasma concentration-time curves in rhesus monkeys after the first intravenous and intravitreal injections of IBI302 at different doses.
FIG. 14 depicts mean plasma concentration-time curves in rhesus monkeys after the last intravenous and intravitreal injections of IBI302 at different doses.
FIG. 15 depicts a dose escalation schematic diagram of the phase I clinical study described in Example 5.
FIG. 16 depicts a study flow schematic diagram of the phase I clinical study described in Example 5.
FIG. 17 depicts the trend of change in the number of correctly identified letters and comparison to a baseline as shown in the initial trial results of the phase I clinical study described in Example 5.
FIG. 18 depicts the trend of change in central subfield thickness (CST) and comparison to a baseline (ΔCST) as shown in the initial trial results of the phase I clinical study described in Example 5.
FIG. 19 depicts the changes in BCVA (number of EDTRS letters) examination results for all dose groups in the overall study results of the phase I clinical study described in Example 5 over time, using the last non-missing value before the first administration of the study drug (including the data from the screening period) as a baseline.
FIG. 20 depicts the changes in BCVA (number of EDTRS letters) from a baseline for all dose groups in the overall study results of the phase I clinical study described in Example 5 over time, using the last non-missing value before the first administration of study drug (including the data from the screening period) as a baseline.
The change values are defined as the mean value of the difference between the measurement at each visit and the baseline.
FIG. 21 depicts the changes in central subfield thickness by ophthalmic OCT examination for all dose groups in the overall study results of the phase I clinical study described in Example 5 over time, using the last non-missing value before the first administration of the study drug (including the data from the screening period) as a baseline.
FIG. 22 depicts the changes in central subfield thickness by ophthalmic OCT examination from a baseline for all dose groups in the overall study results of the phase I clinical study described in Example 5 over time, using the last non-missing value before the first administration of study drug (including the data from the screening period) as a baseline. The change values are defined as the mean value of the difference between the measurement at each visit and the baseline.

### DETAILED DESCRIPTION

The present invention will be further illustrated below with reference to examples, and the following examples should not be construed as limiting the present invention.

### Abbreviations

- AMD: Age-related macular degeneration
- BCVA: Best corrected visual acuity
- OCT: Optical coherence tomography
- CNV: Choroidal neovascularization
- AE: Adverse event
- DLT: Dose-limiting toxicity
- EC50: Median effective concentration
- SAE: Serious adverse event
- TEAE: Treatment emergent adverse event
- CR1: Human complement receptor type 1

### Example 1: In Vitro Pharmacodynamic Study

Affinity for cytokines of the VEGF family and the complement family of IBI302

The affinity for the relevant ligands, i.e., the VEGF family as well as C3b and C4b of IBI302 was investigated using Biacore T200, as shown in Table 1.

The affinity for cytokines of the VEGF family of IBI302 was comparable to that of an aflibercept injection, and was significantly stronger than that of a bevacizumab injection. The affinity for C4b of IBI302 was comparable to that of CR1; the affinity for C3b of IBI302 was slightly weaker than that of CR1, probably due to the fact that the CID end of IBI302 was only a part of the domain of CR1, but it still reached the nM level.

**Table 1. Affinity for cytokines of the VEGF family or the complement family of IBI302 and three controls**

| Test drugs | Ligands | Ka×E5(M-1s-1) | Kd×E-5 (s-1) | KD |
|---|---|---|---|---|
| IBI302 | VEGF-A165 | 187 | 8.75 | 4.68 pM |
| | VEGF-A121 | 85.7 | 5.79 | 6.76 pM |
| | PlGF | 10.0 | 179.7 | 1.79 nM |
| | C3b | 2.88 | 287.0 | 9.96 nM |
| | C4b | 4.32 | 334.9 | 7.75 nM |
| Bevacizumab injection | VEGF-A165 | 12.62 | 4.06 | 32.2 pM |
| | VEGF-A121 | 3.75 | 2.49 | 66.2 pM |
| | PlGF | NB | NB | NB |
| Aflibercept injection | VEGF-A165 | 205 | 7.93 | 3.87 pM |
| | VEGF-A121 | 54.6 | 4.25 | 7.78 pM |
| | PlGF | 33.2 | 384.5 | 1.16 nM |
| CR1 | C3b | 31.6 | 109.7 | 0.35 nM |
| | C4b | 0.47 | 80.5 | 17.1 nM |

| | | | | |
|---|---|---|---|---|
| NB: not detected. | | | | |

### Inhibitory effect of IBI302 on the VEGF-A-induced HUVEC proliferation

After the reaction of IBI302 at a series of concentration gradients with VEGF-A165 at a fixed concentration, HUVEC cells showed different proliferation capacities along with different amounts of free VEGF-A165, and the number of living HUVEC cells was determined using a CCK-8 color development kit, so that the inhibition efficiency of IBI302 on the proliferation of HUVEC cells was determined. Meanwhile, the aflibercept injection and the bevacizumab injection were used as controls.

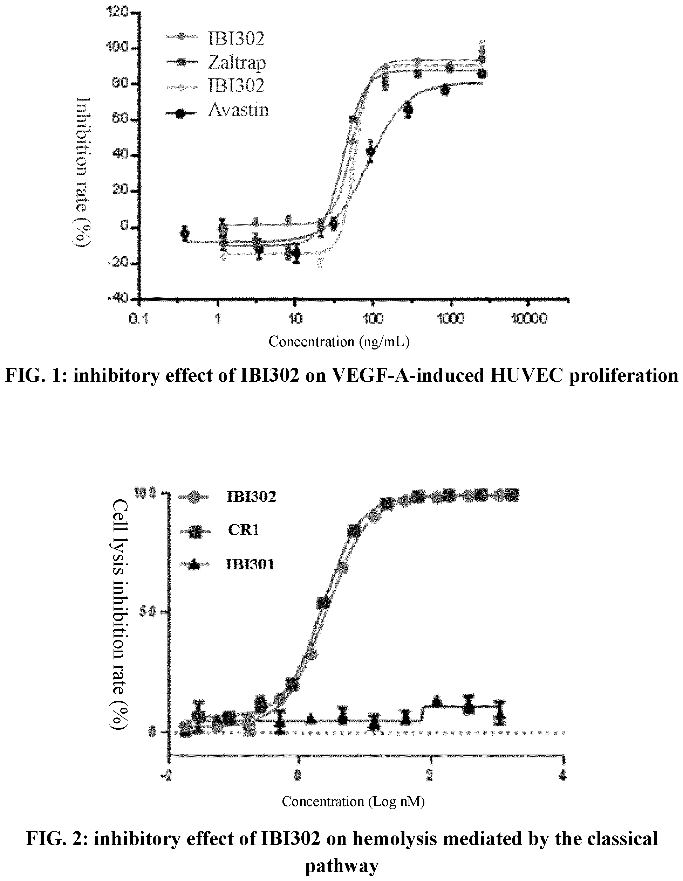

The results showed that: the median effective concentrations (EC50) of IBI302 were 54.36 ng/mL and 57.53 ng/mL, equivalent to 347 pmol/L and 368 pmol/L, respectively; the EC50 of the aflibercept injection was 41.02 ng/mL, equivalent to 357 pmol/L; and the EC50 of the bevacizumab injection was 87.07 ng/mL, equivalent to 584 pmol/L. IBI302 could significantly inhibit VEGF-induced HUVEC proliferation, and had activity similar to aflibercept and slightly stronger than the bevacizumab injection, as shown in Table 2 and FIG. 1.

**Table 2. Inhibitory effect of IBI302 on VEGF-A-induced HUVEC proliferation**

| Sample | IBI302^{∗} | Aflibercept injection | IBI302^{∗} | Bevacizumab injection |
|---|---|---|---|---|
| EC50 (ng/mL) | 54.36 | 41.02 | 57.53 | 87.07 |

| | | | | |
|---|---|---|---|---|
| ^{∗}Since the test was performed on two plates, IBI302 was tested twice. | | | | |

### Inhibitory effect of IBI302 on a classical pathway

A human serum complement was adopted to stimulate a sensitized sheep red blood cell model, and IBI302 can inhibit the hemolytic reaction of the sensitized sheep red blood cells induced by the human serum complement in a concentration-dependent manner within a concentration range of 0.02-1080.26 nM, with a median effective concentration (EC50) value of 2.514 nM. The EC50 value of CR1 was 2.254 nM. This suggests that IBI302 can significantly inhibit the classical pathway, similar to CR1. The specific results are shown in FIG. 2.

### Inhibitory effect of IBI302 on an alternative pathway

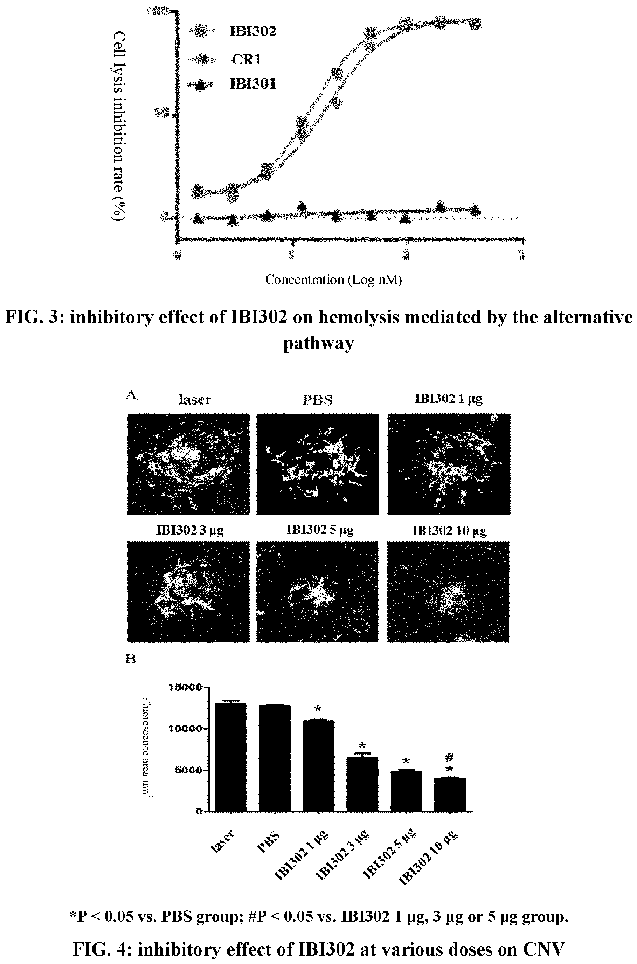

A human serum complement was used to stimulate a rabbit red blood cell model, and IBI302 can inhibit the hemolytic reaction of the rabbit red blood cells induced by the human serum complement in a concentration-dependent manner within a concentration range of 1.50-383.39 nM, with an EC50 value of 14.59 nM. The EC50 value of CR1 was 15.30 nM. This suggests that IBI302 can significantly inhibit the alternative pathway, similar to CR1. The specific results are shown in FIG. 3.

### Effect of IBI302 on barrier function of the complement-activated oxidatively damaged hRPE cells and a mechanism thereof

In the experiment, hRPE cells were adopted and divided into a normal group (nor), a model group (ctr), an aflibercept group (VEGF-Trap), a CR1 group and an IBI302 group. t-BHP and 10% normal human serum were added to all groups except the normal group, and an equal volume of PBS was added to the normal group. The corresponding drugs were then added to the treatment groups, respectively, to reach a final concentration of 1 µg/mL, and an equal volume of PBS was added to the normal group and the model group. After 4 h of culture, the barrier function of the hRPE monolayer cells was tested. After 4 h of culture, VEGF, chemokine (C-C motif) ligand 2 (CCL2), C3a, C5a and MAC secreted by hRPE cells were detected by ELISA method.

The transepithelial resistance (TER) values of complement-activated oxidatively damaged hRPE cells in the model group (ctr), the aflibercept group, the CR1 group and the IBI302 group were 54.01%, 64.64%, 63.48% and 75.90% of the TER value of hRPE cells in the normal group (nor), respectively. The TER value was significantly increased in the aflibercept group, the CR1 group and the IBI302 group as compared to that in the model group, and the TER value in IBI302 group was also significantly different from that in the aflibercept group and the CR1 group, as shown in Table 3.

**Table 3. Effect of IBI302 on transepithelial resistance values**

| Groups | Transepithelial resistance value (Ω cm²) | | | Mean±SD |
|---|---|---|---|---|
| nor | 58.70 | 58.97 | 59.80 | 59.16±0.57 |
| ctr | 35.82 | 30.87 | 29.17 | 31.95±3.45 |
| Aflibercept | 38.81 | 38.57 | 37.34 | 38.24±0.79# |
| CR1 | 38.19 | 35.23 | 39.24 | 37.55±2.08#^{∗} |
| IBI302 | 45.19 | 43.37 | 46.14 | 44.90±1.41^{∗} |

| | | | | |
|---|---|---|---|---|
| ^{∗}P < 0.05 vs. model group; #P < 0.05 vs. IBI302 group. | | | | |

The concentration of VEGF secreted by hRPE cells was significantly reduced in the aflibercept group, the CR1 group and the IBI302 group as compared to that in the model group. The concentration of VEGF was significantly reduced in the group IBI302 as compared to that in the aflibercept group and the CR1 group. The concentration of CCL2 secreted by hRPE cells was significantly reduced in the CR1 group and the IBI302 group as compared to that in the model group; while there was no significant statistical difference in CLL2 secreted by hRPE cells between the aflibercept group and the model group. The concentration of CCL2 was significantly reduced in the IBI302 group as compared to that in the aflibercept group and the CR1 group, as shown in Tables 4 and 5.

**Table 4. Effect of IBI302 on the expression of VEGF**

| Groups | VEGF concentration (pg/mg) | | | Mean±SD |
|---|---|---|---|---|
| ctr | 79.49 | 82.18 | 88.29 | 83.32±4.51 |
| Aflibercept | 59.39 | 68.28 | 64.91 | 64.19±4.49#^{∗} |
| CR1 | 74.48 | 75.49 | 74.24 | 74.74±0.66#^{∗} |
| IBI302 | 56.24 | 50.28 | 50.16 | 52.23±3.48^{∗} |

| | | | | |
|---|---|---|---|---|
| ^{∗}P < 0.05 vs. model group; #P < 0.05 vs. IBI302 group. | | | | |

**Table 5. Effect of IBI302 on the expression of CCL2**

| Groups | CCL2 concentration (pg/mg) | | | Mean±SD |
|---|---|---|---|---|
| ctr | 43.15 | 56.72 | 59.37 | 53.08±8.70 |
| Aflibercept | 50.38 | 44.19 | 53.28 | 49.28±4.46# |
| CR1 | 42.69 | 39.72 | 39.47 | 40.63±1.79#^{∗} |
| IBI302 | 26.37 | 22.16 | 24.38 | 24.30±2.11^{∗} |

| | | | | |
|---|---|---|---|---|
| ^{∗}P < 0.05 vs. model group; #P < 0.05 vs. IBI302 group. | | | | |

The experimental results for the effect of hRPE complement activation indicated that: both the CR1 group and the IBI302 group significantly reduced the expression of complement active ingredients C3a, C5a and MAC as compared to the model group (ctr). There was no statistical difference in the inhibition on the expression of C3a, C5a and MAC between the IBI302 group and the CR1 group. In addition, there was no statistical difference in the effect on the generation of the complement active ingredients C3a, C5a and MAC between the aflibercept group and the model group, as shown in Tables 6, 7 and 8.

**Table 6. Effect of IBI302 on the expression of C3a**

| Groups | C3a concentration (ng/mg) | | | Mean±SD |
|---|---|---|---|---|
| ctr | 93.25 | 95.26 | 88.63 | 92.38±3.40 |
| Aflibercept | 95.81 | 85.29 | 93.07 | 91.39±5.46 |
| CR1 | 71.49 | 71.16 | 68.07 | 70.24±1.89^{∗} |
| IBI302 | 73.28 | 71.05 | 71.79 | 72.04±1.14^{*} |

| | | | | |
|---|---|---|---|---|
| ^{∗}P < 0.05 vs. model group. | | | | |

**Table 7. Effect of IBI302 on the expression of C5a**

| Groups | C5a concentration (ng/mg) | | | Mean±SD |
|---|---|---|---|---|
| ctr | 23.59 | 30.67 | 22.18 | 25.48±4.55 |
| Aflibercept | 22.18 | 27.48 | 23.99 | 24.55±3.46 |
| CR1 | 19.38 | 17.28 | 18.48 | 18.38±1.05^{∗} |
| IBI302 | 16.93 | 14.25 | 16.55 | 15.91±1.45^{∗} |

| | | | | |
|---|---|---|---|---|
| ^{∗}P < 0.05 vs. model group. | | | | |

**Table 8. Effect of IBI302 on the generation of MAC**

| Groups | MAC concentration (ng/mg) | | | Mean±SD |
|---|---|---|---|---|
| ctr | 182.56 | 196.47 | 202.13 | 193.72±10.07 |
| Aflibercept | 176.36 | 175.40 | 204.32 | 185.36±16.43 |
| CR1 | 146.37 | 154.46 | 149.92 | 150.25±4.06^{∗} |
| IBI302 | 132.49 | 139.71 | 160.91 | 144.37±14.77^{∗} |

| | | | | |
|---|---|---|---|---|
| ^{∗}P < 0.05 vs. model group. | | | | |

Those experimental results indicated that IBI302 has a protective effect on the barrier function of complement-activated oxidatively damaged hRPE cells possibly by a mechanism of further reducing CCL2 and VEGF secreted by hRPE cells by inhibiting the expression of both VEGF and complement active ingredients C3a, C5a and MAC after the complement activation.

### Example 2. Pharmacodynamic Study in Animals

Study on the effect of IBI302 on laser-induced choroidal neovascularization in mice and a mechanism thereof This experiment was performed using C57BL/6J mice with laser photocoagulation of 4 spots, and the mice included in the study were randomly divided into the following 6 groups with 2 mice (4 eyes) in each group: a laser modeling group, a phosphate buffered saline (PBS) treatment group, an IBI302 1 µg/µL dose group, an IBI302 3 µg/µL dose group, an IBI302 5 µg/µL dose group and an IBI302 10 µg/µL dose group. Immediately after the laser photocoagulation, choroidal neovascularization (CNV) mice in each group were injected with 1 µL of IBI302 at each dose or PBS at the vitreous cavities of both eyes. On day 7 after the laser photocoagulation, fluorescein isothiocyanate-dextran (FITC-Dextran) orbital sinus perfusion and choroidal flat mount were performed to observe the effect of IBI302 at each dose on the area of laser-induced CNV in mice.

The mice were further divided in the following groups: an IBI302 group, an aflibercept (VEGF-Trap) group, a CR1 group and a PBS group. Immediately after the laser photocoagulation, IBI302 and control drugs were both at 10 µg/µL, and the CNV mice in each group were injected with 1 µL of samples at each dose at the vitreous cavities of both eyes. On day 7 after the laser photocoagulation, the fundus angiography was performed in mice to observe the CNV. The mice were anesthetized with chloral hydrate and dilated with compound tropicamide. Firstly, infrared fundus photographs were taken, and then FITC-Dextran orbital sinus perfusion, indocyanine green angiography (ICGA), and fundus fluorescence angiography (FFA) were performed sequentially. Finally, the concentrations of VEGFs, CCL2, TNF-α, C3a and C5a proteins in RPE-choroid were detected by an ELISA method.

### Experimental results:

Inhibition of IBI302 at different doses on CNV: on day 7 after the laser photocoagulation, FITC-Dextran orbital sinus perfusion and choroidal flat mount were performed in mice sequentially, and new vessels were shown to be in a petal-shaped structure. There was no statistical difference in the CNV areas between the laser group and the PBS group. The CNV areas of the IBI302 1 µg group, the IBI302 3 µg group, the IBI302 5 µg group and the IBI302 10 µg group were reduced by 14.27%, 48.79%, 62.46% and 68.83%, respectively, as compared to that of the PBS group. There was statistically significant difference in the CNV areas between the IBI302 10 µg group and the IBI302 1 µg group, the IBI302 3 µg group, or the IBI302 5 µg group, as shown in Table 9 and FIG. 4.

**Table 9. CNV areas after FITC-Dextran orbital sinus perfusion for IBI302 dose groups**

| Groups | | CNV area (µm²) | | | Mean±SD |
|---|---|---|---|---|---|
| laser | 12305.34 | 12957.21 | 13048.31 | 13442.94 | 12938.45±471.79 |
| PBS | 12614.93 | 12901.45 | 12835.31 | 12518.08 | 12717.44±180.74 |
| IBI302 1 µg | 10846.59 | 11156.42 | 10836.47 | 10771.92 | 10902.85±172.25^{∗} |
| IBI302 3 µg | 6682.38 | 6803.82 | 6823.45 | 5738.39 | 6512.018±519.51^{∗} |
| IBI302 5 µg | 4704.49 | 4592.72 | 4629.02 | 5172.53 | 4774.694±269.28^{∗} |
| IBI302 10 µg | 3837.91 | 3938.81 | 3916.53 | 4163.03 | 3964.072±139.52^{∗}# |

| | | | | | |
|---|---|---|---|---|---|
| ^{∗}P < 0.05 vs. PBS group; #P < 0.05 vs. IBI302 1 µg, 3 µg or 5 µg group. | | | | | |

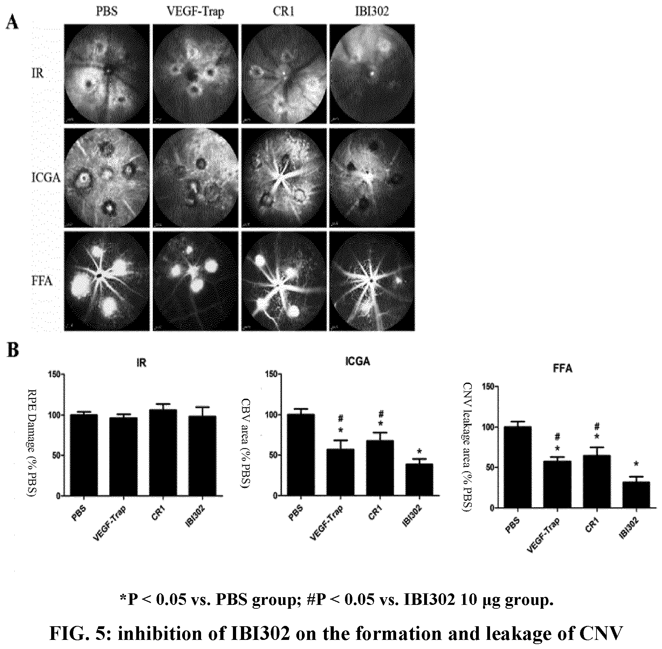

IBI302 at 10 µg/eye, aflibercept and CR1 all inhibited the area and leakage of CNV as compared to the PBS control group. IBI302 at 10 µg/eye could significantly inhibit the area and leakage of CNV as compared to the aflibercept and CR1 groups, as shown in Tables 10 and 11 and FIG. 5.

**Table 10. Percentage of leakage area of each group in FFA**

| Groups | Percentage of leakage area in FFA | | | | Mean±SD |
|---|---|---|---|---|---|
| PBS | 100.00 | 100.00 | 100.00 | 100.00 | 100±6.80 |
| Aflibercept | 63.17 | 50.49 | 56.28 | 58.94 | 57.22±5.31^{∗}# |
| CR1 | 67.31 | 69.52 | 71.64 | 48.89 | 64.34±10.45^{∗}# |
| IBI302 10 µg | 31.09 | 35.72 | 37.14 | 21.37 | 31.33±7.12^{∗} |

| | | | | | |
|---|---|---|---|---|---|
| ^{∗}P < 0.05 vs. PBS group; #P < 0.05 vs. IBI302 10 µg group. | | | | | |

**Table 11. Percentage of CNV area of each group in ICGA**

| Groups | Percentage of CNV area in ICGA | | | | Mean±SD |
|---|---|---|---|---|---|
| PBS | 100.00 | 100.00 | 100.00 | 100.00 | 100±7.00 |
| Aflibercept | 68.31 | 64.69 | 48.28 | 44.72 | 56.50±11.73^{∗}# |
| CR1 | 73.17 | 67.18 | 76.28 | 52.89 | 67.38±10.37^{∗}# |
| IBI302 10 µg | 31.09 35.72 | 39.41 | 47.02 | | 38.31±6.73^{∗} |

| | | | | | |
|---|---|---|---|---|---|
| ^{∗}P < 0.05 vs. PBS group; #P < 0.05 vs. IBI302 10 µg group. | | | | | |

Inhibitory effect of IBI302 on the generation of VEGF of the laser-induced CNV mice: on day 3 after the laser photocoagulation, IBI302 at 10 µg/eye, aflibercept and CR1 all could inhibit the generation of VEGF in laser-induced CNV mice as compared to the PBS group. There was statistically significant difference in the inhibition of the generation of VEGF between the IBI302 group and the aflibercept group or the CR1 group, as shown in Table 12.

**Table 12. Effect of IBI302 on the expression of VEGF**

| Groups | VEGF concentration (pg/mg) | | | | Mean±SD |
|---|---|---|---|---|---|
| PBS | 150.54 | 167.26 | 147.27 | 179.37 | 161.11±14.99 |
| Aflibercept | 100.84 | 120.46 | 90.87 | 108.41 | 105.15±12.48^{∗}# |
| CR1 | 120.37 | 123.19 | 113.89 | 129.01 | 121.62±6.28^{∗}# |
| IBI302 10 µg | 76.29 | 72.43 | 66.53 | 72.17 | 71.86±4.02^{∗} |

| | | | | | |
|---|---|---|---|---|---|
| ^{∗}P < 0.05 vs. PBS group; #P < 0.05 vs. IBI302 10 µg group. | | | | | |

Inhibition of IBI302 on the complement activation in the laser-induced CNV mice: on day 3 after the laser photocoagulation, it was found that IBI302 at 10 µg/eye could significantly inhibit the deposition of MAC in the choroid as compared to the PBS group, the aflibercept group and the CR1 group by immunofluorescence of choroidal flat mount. 12 h after the laser photocoagulation, expression levels of C3a and C5a in the RPE-choroid complex were detected by an ELISA method, and it was found that the expression levels of C3a and C5a in the CR1 group and the IBI302 group were significantly reduced as compared to those in the model group. There was no statistical difference in the concentrations of C3a and C5a between the IBI302 group and the CR1 group; and the concentrations of C3a and C5a in the IBI302 group were significantly reduced as compared to those in the aflibercept group, as shown in Tables 13 and 14.

**Table 13. Effect of IBI302 on the expression of C3a**

| Groups | C3a concentration (ng/mg) | | | Mean±SD |
|---|---|---|---|---|
| PBS | 8.97 | 9.74 | 10.98 | 9.90±1.01 |
| Aflibercept | 8.59 | 9.30 | 11.00 | 9.63±1.24# |
| CR1 | 6.59 | 6.95 | 5.58 | 6.37±0.71^{∗} |
| IBI302 10 µg | 5.96 | 5.87 | 5.75 | 5.86±0.11^{∗} |

| | | | | |
|---|---|---|---|---|
| ^{∗}P < 0.05 vs. PBS group; #P < 0.05 vs. IBI302 10 µg group. | | | | |

**Table 14. Effect of IBI302 on the expression of C5a**

| Groups | C5a concentration (ng/mg) | | | Mean±SD |
|---|---|---|---|---|
| PBS | 5.54 | 3.25 | 4.37 | 4.39±1.15 |
| Aflibercept | 4.75 | 4.71 | 5.31 | 4.92±0.34# |
| CR1 | 2.67 | 2.53 | 3.32 | 2.84±0.42^{∗} |
| IBI302 10 µg | 3.16 | 2.47 | 2.44 | 2.69±0.41^{∗} |

| | | | | |
|---|---|---|---|---|
| ^{∗}P < 0.05 vs. PBS group; #P < 0.05 vs. IBI302 10 µg group. | | | | |

Inhibitory effect of IBI302 on inflammatory factors CCL2 and TNF-α in the laser-induced CNV mice: the expression levels of inflammatory factors CCL2 and TNF-α in the RPE-choroidal complex were measured by ELISA at 12 h and on day 3 after the laser photocoagulation, respectively. The expression of inflammatory factors CCL2 and TNF-α in the CR1 and IBI302 10 µg/eye groups was significantly reduced as compared to that in the PBS group. The concentrations of CCL2 and TNF-α in the IBI302 group were significantly lower than those in the aflibercept group, as shown in Tables 15 and 16.

**Table 15. Effect of IBI302 on the expression of CCL2**

| Groups | CCL2 concentration (pg/mg) | | | | Mean±SD |
|---|---|---|---|---|---|
| PBS | 238.29 | 219.25 | 211.48 | 190.82 | 214.96±17.01 |
| Aflibercept | 219.27 | 217.45 | 218.45 | 184.93 | 210.02±16.75# |
| CR1 | 160.94 | 178.29 | 171.38 | 183.01 | 173.41±9.58^{∗} |
| IBI302 10 µg | 140.28 145.28 | 143.14 | 193.15 | | 155.46±25.21^{∗} |

| | | | | | |
|---|---|---|---|---|---|
| ^{∗}P < 0.05 vs. PBS group; #P < 0.05 vs. IBI302 10 µg group. | | | | | |

**Table 16. Effect of IBI302 on the expression of TNF-α**

| Groups | TNF-α concentration (pg/mg) | | | | Mean±SD |
|---|---|---|---|---|---|
| PBS | 259.19 | 210.48 | 205.01 | 203.30 | 219.50±23.07 |
| Aflibercept | 214.18 | 209.25 | 203.56 | 200.34 | 206.83±6.13# |
| CR1 | 148.94 | 157.94 | 169.23 | 159.3 | 158.85±8.31^{∗} |
| IBI302 10 µg | 130.46 | 159.35 | 134.56 | 125.59 | 137.49±15.03^{∗} |

| | | | | | |
|---|---|---|---|---|---|
| ^{∗}P < 0.05 vs. PBS group; #P < 0.05 vs. IBI302 10 µg group. | | | | | |

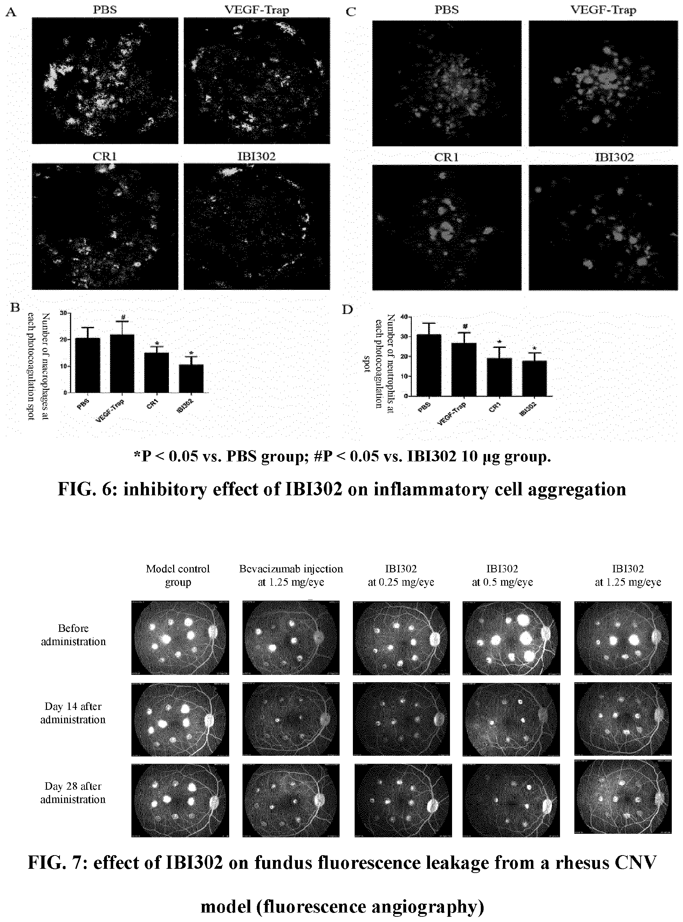

Inhibitory effect of IBI302 on the aggregation of inflammatory cells in the laser-induced CNV mice: the aggregations of neutrophils and macrophages in the choroid were detected by immunofluorescence of choroidal flat mount on days 1 and 3 after the laser photocoagulation. IBI302 could significantly inhibit the depositions of neutrophils and macrophages in the choroid, and there was statistical difference between the IBI302 group and the PBS group or the aflibercept group, as shown in Tables 17 and 18 and FIG. 6.

**Table 17. Effect of IBI302 on the aggregation of neutrophils**

| Groups | Number of neutrophils/laser spots | | | | | Mean±SD |
|---|---|---|---|---|---|---|
| PBS | 37 | 28 | 29 | 23 | 37 | 30.80±6.10 |
| Aflibercept | 24 | 26 | 21 | 28 | 35 | 26.80±5.26# |
| CR1 | 24 | 17 | 26 | 13 | 15 | 19.00±5.70^{∗} |
| IBI302 10 µg | 17 | 19 | 24 | 15 | 13 | 17.60±4.22^{∗} |

| | | | | | | |
|---|---|---|---|---|---|---|
| ^{∗}P < 0.05 vs. PBS group; #P < 0.05 vs. IBI302 10 µg group. | | | | | | |

**Table 18. Effect of IBI302 on the aggregation of macrophages**

| Groups | Number of macrophages/laser spots | | | | | Mean±SD |
|---|---|---|---|---|---|---|
| PBS | 24 | 19 | 15 | 19 | 25 | 20.40±4.10 |
| Aflibercept | 17 | 16 | 26 | 27 | 23 | 21.80±5.07# |
| CR1 | 15 | 16 | 16 | 11 | 17 | 15.00±2.35^{∗} |
| IBI302 10 µg | 13 | 6 | 14 | 9 | 10 | 10.40±3.21^{∗} |

| | | | | | | |
|---|---|---|---|---|---|---|
| ^{∗}P < 0.05 vs. PBS group; #P < 0.05 vs. IBI302 10 µg group. | | | | | | |

The results indicated that IBI302 can inhibit the formation and leakage of the laser-induced CNV in mice possibly by a mechanism of inhibiting the infiltration of macrophages and neutrophils, and thus inhibiting the VEGF secretion and neovascularization by inhibiting the expression of both VEGF and active ingredients C3a, C5a, MAC and CCL2 and TNF-α after the complement activation.

### Experiment on the inhibition of IBI302 on the laser-induced rhesus monkey choroidal neovascularization

In this experiment, laser photocoagulation was performed around the macular fovea of the fundus of the rhesus monkey to induce the choroidal neovascularization in the fundus and establish an animal model similar to the human choroidal neovascularization. 25 monkeys (male and female) with both eyes successfully modeled were selected and divided into the following 5 groups with five monkeys (male and female) in each group: a model control group, a bevacizumab injection 1.25 mg/eye group, an IBI302 0.25 mg/eye group, an IBI302 0.5 mg/eye group and an IBI302 1.25 mg/eye group, as shown in Table 19. On day 21 after the photocoagulation, monkeys in each group were intravitreally injected with the corresponding drugs in a single dose of 50 µL/eye.

Color fundus photograhpy and fundus fluorescence angiography were performed on days 14 and 28 after the administration, and optical coherence tomography (OCT) was performed on days 11 and 25 after the administration to observe the inhibition of the test sample on the choroidal neovascularization. On day 29 after the administration, the aqueous humor was extracted from both eyes of the monkey for VEGF detection, the left eye was taken for the histopathological examination, and the right eye was taken for the immunohistochemical staining examination of CD31 and C5b-9.

**Table 19. Grouping and administration design**

| Groups | Route of administration | Administration dosage mg/eye | Administration concentration mg/mL | Administration volume µL/eye | Number of animals (animal) | Number of eyeballs |
|---|---|---|---|---|---|---|
| Model control group | Intravitreal injection | - | - | 50 | 5 | 10 |
| Bevacizumab injection 1.25 mg/eye group | Intravitreal injection | 1.25 | 25 | 50 | 5 | 10 |
| IBI302 0.25 mg/eye group | Intravitreal injection | 0.25 | 5 | 50 | 5 | 10 |
| IBI302 0.5 mg/eye group | Intravitreal injection | 0.5 | 10 | 50 | 5 | 10 |
| IBI302 1.25 mg/eye group | Intravitreal injection | 1.25 | 25 | 50 | 5 | 10 |

### The results showed that:

Fluorescence angiography and OCT examination: in the model control group, there was no significant change in the fluorescein leakage degree, and no significant reduction in the area of fluorescent spots on days 14 and 28 after the administration, and the fluorescein leakage even tended to increase over time in a part of monkey eyes. No significant change in the number of grade 4 fluorescent spots was observed as compared to that before the administration. On days 11 and 25 after the administration, the OCT examination showed that the retinal thickness was not significantly reduced, the local Bruch's membrane structure was not repaired, and choroidal neovascularization was still evident, with highly reflective light masses.

The leakage area of the bevacizumab injection group on days 14 and 28 after the administration was significantly reduced as compared to that before the administration, and was significantly different from that of the model control group (P < 0.05), and the number of grade 4 fluorescent spots was significantly reduced as compared to that before the administration. On days 11 and 25 after the administration, the OCT examination showed that the retinal thickness at the highest part of all eyeball lesions was significantly reduced, even approached or reached the level before the modeling, and had statistically significant difference as compared to the control group (P < 0.05).

the leakage area of monkeys in the IBI302 0.25 mg/eye group, the IBI302 0.5 mg/eye group and the IBI302 1.25 mg/eye group on days 14 and 28 after the administration was significantly reduced as compared to that before the administration, and was significantly different from that in the model control group (P < 0.05), and the improvement rate of the leakage area of monkeys on day 28 after the administration was also significantly higher than that in the positive control group (P < 0.05). The number of grade 4 fluorescent spots was significantly reduced as compared to that before the administration. On days 11 and 25 after the administration, the OCT examination showed that the retinal thickness was significantly reduced, even approached or reached the level before the modeling, and had statistically significant difference as compared to the model control group (P < 0.05), and no statistically significant difference in each index as compared to the positive control group (P > 0.05). The laser spot fluorescein leakage, the number of fluorescence spots and the retinal thickness of the fundus of monkeys of each group before and after the administration are shown in Tables 20, 21 and 22 and FIG. 7, respectively.

**Table 20. Effect of IBI302 on the reduction (mm²) and improvement rate of the fluorescein leakage area in rhesus monkeys**

| Index measurement time | Model control group | Bevacizumab injection 1.25 mg/eye group | IBI302 0.25 mg/eye group | IBI302 0.5 mg/eye group | IBI302 1.25 mg/eye group |
|---|---|---|---|---|---|
| Reduction of fluorescein leakage area (mm²) | | | | | |
| Day 14 after the administration | 0.952 ± 9.211 | 20.834 ± 20.279 ^{∗} | 23.715 ± 11.349 ^{∗} | 21.670 ± 11.650 ^{∗} | 23.177 ± 16.198 ^{∗} |
| Day 28 after the administration | 3.270 ± 9.918 | 17.209 ± 20.766 ^{∗} | 22.685 ± 11.910 ^{∗} | 19.742 ± 11.197 ^{∗} | 23.888 ± 13.939 ^{∗} |

| Improvement rate of fluorescein leakage area (%) | | | | | |
|---|---|---|---|---|---|
| Day 14 after the administration | -9.015 ± 49.323 | 68.728 ± 16.744 ^{∗} | 80.684 ± 9.881 ^{∗} | 82.625 ± 12.904 ^{∗} | 73.854 ± 13.774 ^{∗} |
| Day 28 after the | 11.739 ± 36.270 | 47.656 ± 25.359 ^{∗} | 75.556±13.108 ^{∗}# | 73.577 ± 16.676 ^{∗}# | 79.919±13.825 ^{∗}# |

| Index measurement time | Model control group | Bevacizumab injection 1.25 mg/eye group | IBI302 0.25 mg/eye group | IBI302 0.5 mg/eye group | IBI302 1.25 mg/eye group |
|---|---|---|---|---|---|
| administration | | | | | |

| | | | | | |
|---|---|---|---|---|---|
| ^{∗}P ≤ 0.05 vs. model control group; #P ≤ 0.05 vs. positive control group. | | | | | |

**Table 21. Effect of IBI302 on fluorescent spot grade in rhesus monkeys**

| Measurement time Grade | Model control group | Bevacizumab injection 1.25 mg/eye group | IBI302 0.25 mg/eye group | IBI302 0.5 mg/eye group | IBI302 1.25 mg/eye group |
|---|---|---|---|---|---|
| Before administration | | | | | |
| Grade 3 | 17 | 19 | 20 | 20 | 20 |
| Grade 4 | 40 | 38 | 41 | 38 | 38 |
| Day 14 after the administration | | ^{∗} | ^{∗} | ^{∗} | ^{∗} |
| Grade 3 | 8 | 19 | 10 | 12 | 26 |
| Grade 4 | 45 | 9 | 4 | 4 | 7 |
| Day 28 after the administration | | ^{∗} | ^{∗} | ^{∗} | ^{∗} |
| Grade 3 | 12 | 22 | 13 | 17 | 21 |
| Grade 4 | 38 | 9 | 6 | 5 | 6 |

| | | | | | |
|---|---|---|---|---|---|
| ^{∗}P ≤ 0.05 vs. model control group. | | | | | |

**Table 22. Effect of IBI302 on reduction and improvement rate of retinal thickness in rhesus monkeys**

| Index measurement time | Model control group | Bevacizumab injection 1.25 mg/eye group | IBI302 0.25 mg/eye group | IBI302 0.5 mg/eye group | IBI302 1.25 mg/eye group |
|---|---|---|---|---|---|
| Reduction of retinal thickness (µm) | | | | | |
| Day 14 after the administration | 36 ± 50 | 172 ± 69^{∗} | 185 ± 98^{∗} | 136 ± 64^{∗} | 193 ± 120^{∗} |
| Day 28 after the administration | 48 ± 72 | 198 ± 90^{∗} | 192 ± 101^{∗} | 146 ± 57^{∗} | 199 ± 110^{∗} |

| Improvement rate of retinal thickness (%) | | | | | |
|---|---|---|---|---|---|
| Day 14 after the administration | 18.05 ± 22.17 | 80.46 ± 19.91^{∗} | 90.04 ± 13.89 ^{∗} | 86.03 ± 20.62^{∗} | 87.67 ± 14.91^{∗} |
| Day 28 after the administration | 23.07 ± 33.14 | 89.70 ± 17.56^{∗} | 94.23 ± 12.78 ^{∗} | 94.00 ± 19.02^{∗} | 92.79 ± 13.98^{∗} |

| | | | | | |
|---|---|---|---|---|---|
| ^{∗}P ≤ 0.05 vs. model control group. | | | | | |

VEGF detection: there was 1 monkey with the VEGF concentration in the serum measured around the lower limit of quantitation on days 14 and 28 after the administration, but lower than the lower limit of quantitation at other time points in each of the bevacizumab injection 1.25 mg/eye group and the model control group. On day 29 after the administration, the VEGF concentration in aqueous humor of the monkeys in the bevacizumab injection 1.25 mg/eye group was reduced as compared to that in the control group, with statistically significant difference (P < 0.05), and the VEGF concentration in aqueous humor of the monkeys of each of the IBI302 groups was lower than the lower limit of quantitation. The specific results are shown in Table 23.

**Table 23. Effect of IBI302 on the VEGF concentration in serum and aqueous humor of rhesus monkeys (pg/mL)**

| Index measurement time | Model control group (n) | Bevacizumab injection 1.25 mg/eye group (n) | IBI302 0.25 mg/eye group (n) | IBI302 0.5 mg/eye Group (n) | IBI302 1.25 mg/eye group (n) |
|---|---|---|---|---|---|
| VEGF in serum | | | | | |
| Before photocoagulation | - | - | - | - | - |
| Before administration | - | - | - | - | - |
| Day 14 after the administration | - | 36.547(1) | - | - | - |
| Day 28 after the administration | 31.411(1) | - | - | - | - |
| VEGF in aqueous humor | | | | | |
| Day 29 after the administration | 94.203 ± 17.360 (10) | 38.644 ± 6.521^{∗} (7) | - | - | - |

- BLOQ (below the limit of quantitation) cannot be calculated; ^{∗}P ≤ 0.05 vs. model control group. Histopathology and immunohistochemistry: HE staining results: the model control group showed minimal-to-moderate focal retinal/choroidal fibroplasia and minimal-to-mild focal neuroepithelial detachment. The severity of the above lesions in each of the IBI302 groups and the bevacizumab injection 1.25 mg/eye group was lower than that in the model control group.

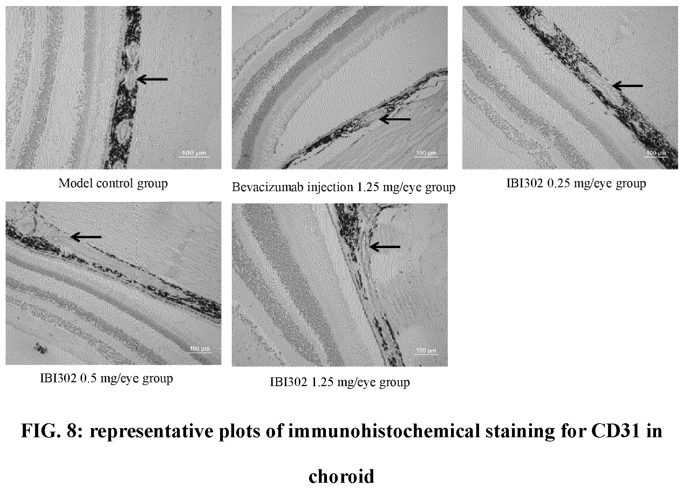

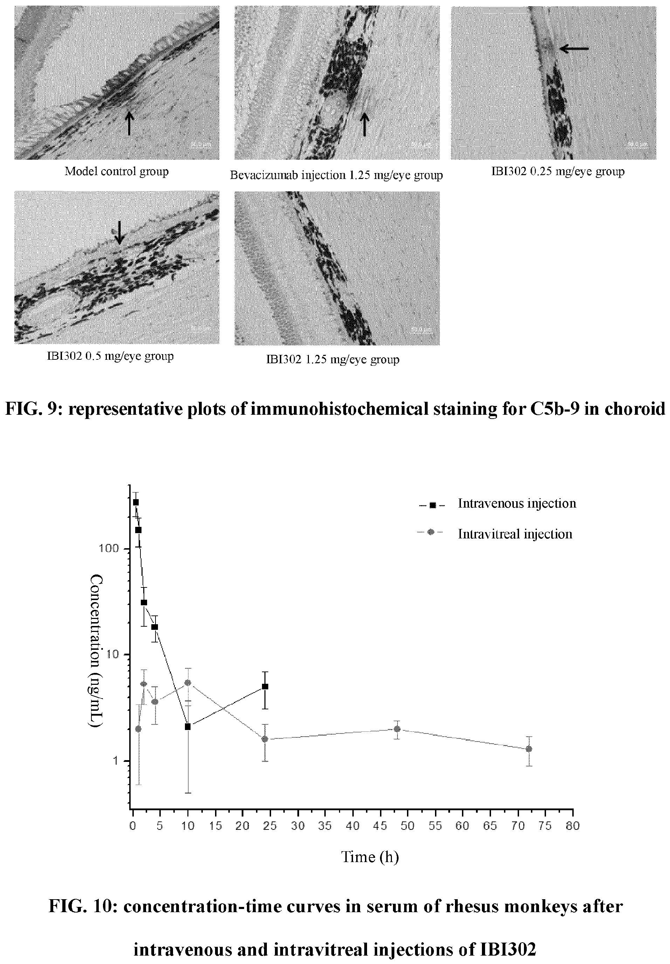

Masson trichrome staining results: the model control group was slightly positive for the minimal retinal focal collagen fibroplasia. There was no significant difference in the positive strength of fibroplasia between the bevacizumab injection 1.25 mg/eye group or each of the IBI302 groups and the model control group; for the positive distribution, the positive control group and each of the IBI302 groups were reduced as compared to the model control group, and no positive staining was observed in one eyeball of the IBI302 1.25 mg/eye group. Immunohistochemical staining results: each of the IBI302 dose groups could reduce the expression of CD31 and C5b-9 in choroid in a certain dose-dependent manner, and the bevacizumab injection had a significant inhibitory effect on the expression of CD31 but had no effect on the expression of C5b-9. The specific results are shown in Tables 24 and 25 and FIGs. 8 and 9.

**Table 24. Immunohistochemical staining results for CD31 in choroid**

| Groups | Staining results | | | | |
|---|---|---|---|---|---|
| | Positive | | Weakly positive | | Negative |
| Model control group | 4 | | 1 | | 0 |
| Bevacizumab injection 1.25 mg/eye group | 0 | | 4 | | 1 |
| IBI302 low-dose group | 1 | | 3 | | 1 |
| IBI302 medium-dose group | 1 | | 3 | | 1 |
| IBI302 high-dose group | 0 | | 3 | | 2 |

**Table 25. Immunohistochemical staining results for C5b-9 in choroid**

| Groups | Staining results | |
|---|---|---|
| | Weakly positive | Negative |
| Model control group | 3 | 2 |
| Bevacizumab injection 1.25 mg/eye group | 3 | 2 |
| IBI302 low-dose group | 2 | 3 |
| IBI302 medium-dose group | 2 | 3 |
| IBI302 high-dose group | 1 | 4 |

The above results indicated that in the laser-induced rhesus monkey choroidal neovascularization model, IBI302 can significantly reduce the fluorescein leakage, the area of fluorescent spots and the number of fluorescent spots at the photocoagulation site, reduce the retinal thickness of at fluorescent spot lesions, restore the integrity and continuity of pigment epithelium, reduce the VEGF level in aqueous humor, relieve fibroplasia and inhibit the expression of CD31 and C5b-9 in choroid, and IBI302 has better efficacy than bevacizumab at a clinical dose.

### Safe pharmacology

50 rhesus monkeys were randomly divided into the following 5 groups: a control group, an IBI302 intravenous injection 8 mg/monkey group, an IBI302 intravenous injection 0.5 mg/eye group, an IBI302 intravenous injection 2 mg/eye group, and an IBI302 intravenous injection 4 mg/eye group, with 10 animals in each group, half male and half female. The general behaviors, mental state, respiratory state and the like of monkeys in each group were observed every day in the treatment period and the recovery period after the interruption; body temperature was measured once a week; the monkeys were observed for the respiration state and detected for the II-lead electrocardiogram and blood pressure about 6 h, 24 h, 48 h and 120 h after the first dose, about 6 h after the last dose, at the week 4 of the interruption period and at the end of the interruption period.

The experimental results indicated that during the treatment period and the recovery period after the interruption, the rhesus monkeys in each group had good general conditions and normal autonomous activities, and had no ethological abnormalities related to the test samples; no abnormalities in body temperature were observed in the rhesus monkeys in each group during the study. There were no drug-related abnormal changes in the heart rate, QT and corrected QT interval, PR interval, RR interval, P wave time, QRS wave time and other electrocardiogram indexes, the systolic pressure, the diastolic pressure, the mean arterial pressure and respiratory status observed by naked eyes in monkeys in each of the IBI302 groups 6 h, 24 h, 48 h and 120 h after the first administration, about 6 h after the last administration, at the week 4 of the recovery period and at the end of the recovery period. This suggests that the intravitreal injection of IBI302 at 0.5 mg/eye, 2 mg/eye or 4 mg/eye or the intravenous injection of IBI302 at 8 mg/monkey has no significant effects on the central nervous system, respiratory system and cardiovascular system in rhesus monkeys.

### Conclusion

### Pharmacodynamic study

IBI302 can specifically bind to the VEGF family through the VID end to inhibit the VEGF-A-induced HUVEC proliferation in an aspect, and can specifically bind to C3b and C4b through the CID end to inhibit the hemolytic reaction mediated by the alternative pathway and the classical pathway in another aspect, indicating that IBI302 is a double-target specific monoclonal antibody with clear target and mechanism of action.

IBI302 plays a role in protecting hRPE cells by inhibiting t-BHP from stimulating the hRPE cells to release VEGF-A, CCL2, C3a, C5a and MAC through double blocking of VEGF-A-mediated and complement-mediated signal pathways; inhibits the laser-induced choroidal neovascularization mice from expressing VEGF, CCL2, TNF-α, C3a, C5a and MAC to relieve the choroidal neovascularization; and inhibits the laser-induced choroidal neovascularization rhesus monkey from expressing VEGF, CD31 and C5-b9 to relieve the choroidal neovascularization and reduce the neovascularization leakage, reduce the retina thickness, and protect pigment epithelium, indicating that IBI302 has definite efficacy.

### Pharmacodynamic characteristics and significance

(1) Double targets: IBI302 specifically binds to VEGF-A165, VEGF-A121 and PIGF through the VID end in an aspect, and specifically binds to C3b and C4b through the CID end in another aspect, suggesting that IBI302 is a double-target medicament and has definite targets.
(2) Clear mechanism of action: the CID of IBI302 can specifically bind to C3b and C4b to inhibit the activation of the classical pathway and the alternative pathway, and relieve the inflammatory reaction mediated by complement activation; the VID end can bind to the VEGF family to block the VEGF-mediated signal pathway, and inhibit the survival and proliferation of vascular endothelial cells, thereby inhibiting angiogenesis, reducing vascular permeability and reducing vascular leakage.
(3) Definite efficacy: in the t-BHP-induced hRPE oxidative stress model, IBI302 has a significant protective effect on hRPE; and in the laser-induced choroidal neovascularization mice and rhesus monkey models, IBI302 can have significant anti-angiogenesis and anti-leakage effects, suggesting that IBI302 has very good clinical efficacy.
(4) Better efficacy than commercially available anti-VEGF drugs: in the t-BHP-induced hRPE oxidative stress model and the laser-induced choroidal neovascularization mice and rhesus monkey models, IBI302 has better efficacy than aflibercept or bevacizumab, suggesting that IBI302 may have better efficacy than commercially available anti-VEGF drugs alone.
(5) Clinical indications: combining the preclinical pharmacological activity of IBI302 to date and the clinical use of similar drugs, IBI302 is clinically proposed for the treatment of wet age-related macular degeneration (AMD).

### Example 3: Pharmacokinetic Study

### Absorption

### Pharmacokinetic study of IBI302 in rhesus monkeys

In a pharmacokinetic study of a single intravenous or intravitreal injection of IBI302 in rhesus monkeys, 27 rhesus monkeys were randomly divided into an intravenous injection group and an intravitreal injection group, of which 21 animals (11 females and 10 males) were in the intravitreal injection group, and 6 animals (3 females, 3 males) were in the intravenous injection group. Blood was collected from 6 animals in each of the intravenous injection group and the intravitreal injection group before the administration and 0.5 h, 1 h, 2 h, 4 h, 10 h, 24 h, 48 h, 72 h, 96 h, 144 h, 192 h, 240 h, 336 h and 504 h after the administration, and serum was isolated. In the intravitreal injection group, aqueous humor, vitreous humor, crystalline lens, cornea, iris, retina, choroid, sclera, optic nerve and serum were taken 4 h, 24 h, 72 h, 168 h, 336 h and 504 h after the administration. The detailed experimental design is shown in Table 26.

**Table 26. Experimental design**

| Groups | Administratio n dosage (mg/eye) | Drug concentration (mg/mL) | Administrati on volume (µL/eye) | Number of animals | Animal No. | | Time of dissection |
|---|---|---|---|---|---|---|---|
| Intravenous injection group | 1 mg/animal | 10 | 0.1 mL/animal | 6 | 1F001 | 1M001 | |
| | | | | | 1F002 | 1M002 | - |
| | | | | | 1F003 | 1M003 | |
| | | | | | 2F001, 2F002 2M001 | | 4 h after the administration |
| | | | | | 2F003, 2M002 2M003 | | 24 h after the administration |
| Intravitreal injection group | 0.5 | 10 | 50 | 21 | 2F004, 2F005 2M004 | | Day 3 after the administration |
| | | | | | 2F006, 2M005 2M006 | | Day 7 after the administration |
| | | | | | 2F007, 2F008 2M007 | | Day 14 after the administration |
| | | | | | 2F009, 2F010 2F011, 2M008 2M009, 2M010 | | Day 21 after the administration |

The results showed that: after intravitreal injection of IBI302, the drug half-life T1/2 was significantly greater than that of intravenous injection. The drug concentration in the ocular tissue was significantly higher than that in serum, with a lower risk of systemic toxicity. In terms of the distribution of the drug in the ocular tissue, the drug was mainly distributed in the vitreous body, retina and choroid of the ocular tissue, and it could remain in the retina and choroid for a long time whether in terms of the terminal elimination half-life T1/2 or the mean residence time MRT, which helped it to take effect.

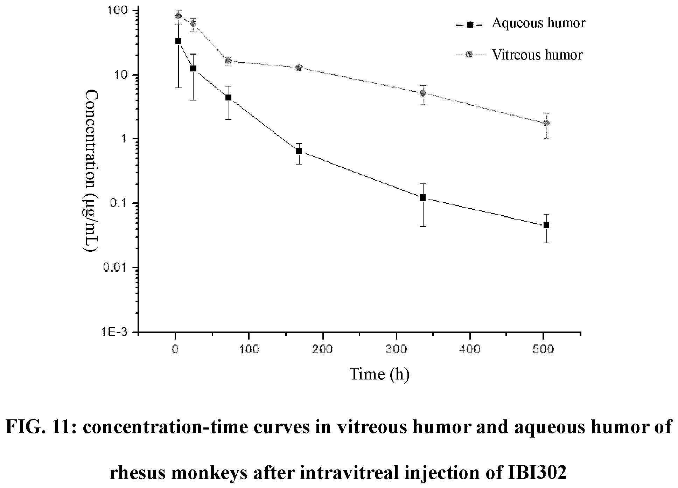

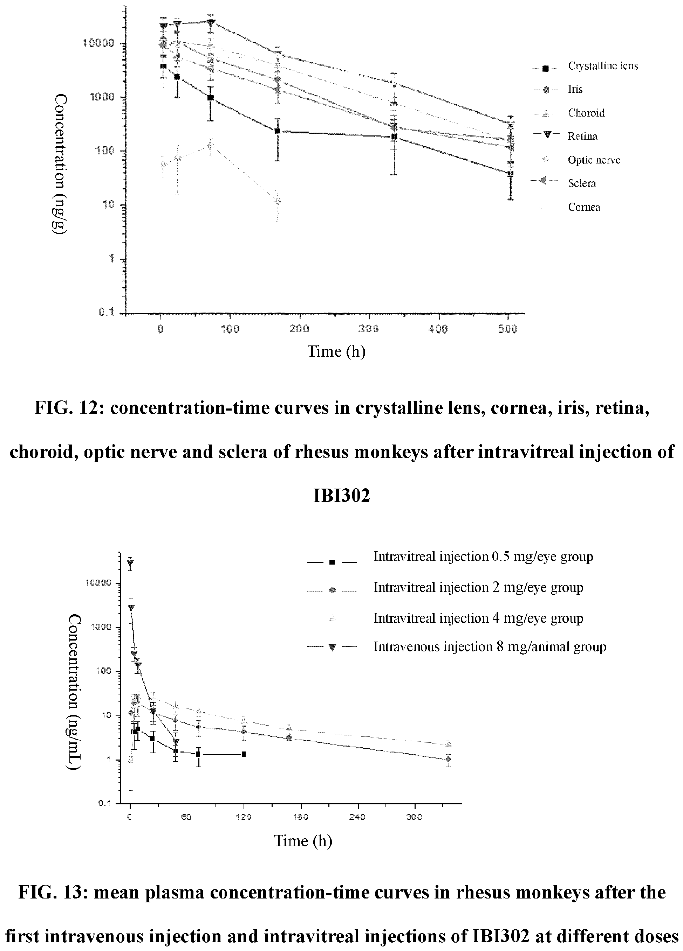

The results of the pharmacokinetics of the serum are shown in Table 27 and FIG. 10, and the results of the pharmacokinetics of the ocular tissue are shown in Table 28 and FIGs. 11 and 12.

**Table 27. Pharmacokinetic parameters for the serum of rhesus monkeys after the intravitreal and intravenous injections of IBI302**

| Parameters | U nit | Intravenous injection (1 mg/animal) | Intravitreal injection (1 mg/animal) |
|---|---|---|---|
| AUC(0-t) | ng·h/mL | 561.7±164.6 | 173.5±45.9 |
| AUC(0-inf) | ng·h/mL | 631.3±161.4 | 252.3±63 |
| MRT(0-t) | h | 3.3±0.8 | 27.2±1.5 |
| Cl | mL/h | 1684.2±478.4 | 4177.9±1037.4 |
| Vd | mL | 23399.6±10614.4 | 247716.1±87020 |
| T1/2 | h | 9.4±1.8 | 41±9.3 |
| Cmax | ng/mL | 238±104.7 | 5.9±2.1 |
| Tmax | h | 0.6±0.2 | 6±4.4 |
| Bioavailability | % | N/A | 30.9 |

| | | | |
|---|---|---|---|
| N/A: not applicable. | | | |

**Table 28. Pharmacokinetic parameters for the ocular tissue in rhesus monkeys after a single intravitreal injection of IBI302**

| Parameters | Unit | Aqueou s humor^{∗} | Vitreous humor^{∗} | Crystallin e lens | Cornea | Iris | Retina | Choroid | Optic nerve | Sclera |
|---|---|---|---|---|---|---|---|---|---|---|
| AUC(0-t) | µg·h/g | 1247.2 | 7030.9 | 261.9 | 1771.4 | 1190.2 | 4065.2 | 1831.4 | 12.9 | 800.6 |
| AUC(0-inf) | µg·h/g | 1253.0 | 7329.4 | 266.7 | 1827.8 | 1209.4 | 4100.7 | 1847.4 | -- | 814.8 |
| MRT | h | 46.3 | 113.6 | 93.5 | 166.2 | 89.1 | 101.4 | 106.5 | 63.5 | 93.8 |
| Cl | mL/h | 0.4 | 0.1 | 1.9 | 0.3 | 0.4 | 0.1 | 0.3 | -- | 0.6 |
| Vd | mL | 50.7 | 11.5 | 242.8 | 35.2 | 48.8 | 13.6 | 28.1 | -- | 74.3 |
| T1/2 | h | 88.1 | 116.4 | 89.8 | 89.1 | 81.8 | 77.3 | 71.9 | -- | 83.9 |
| Cmax | µg/g (µg/mL), | 33.1 | 81.7 | 3.8 | 6.0 | 10.4 | 25.2 | 12.5 | 0.1 | 9.6 |
| Tmax | h | 4.0 | 4.0 | 4.0 | 168.0 | 24.0 | 72.0 | 4.0 | 72 | 4.0 |

### Pharmacokinetic study on repeated administrations of IBI302

In a toxicity study on repeated intravitreal injections of IBI302 in rhesus monkeys for 4 weeks, 50 rhesus monkeys were randomly divided into the following 5 groups: a control group, an IBI302 8 mg/animal intravenous injection group, an IBI302 0.5 mg/eye intravitreal injection group, an IBI302 2 mg/eye intravitreal injection group, and an IBI302 4 mg/eye intravitreal injection group, which were administered once every 2 weeks for 3 consecutive times, with 10 animals in each group, half male and half female. Blood was collected from the control group before and 24 h after the first and last administrations, and serum was prepared. Blood was collected from the IBI302 8 mg/animal intravenous injection group before the first and last administrations, and 5 min, 1 h, 4 h, 8 h, 24 h, 48 h, 72 h, 120 h, 168 h and 336 h after the administrations, and serum was prepared. Blood was collected from the IBI302 intravitreal injection groups before the first and last administrations, and 1 h, 4 h, 8 h, 24 h, 48 h, 72 h, 120 h and 168 h after the administrations, and serum was prepared. The concentration of IBI302 in serum was measured by the established ELISA method. The specific experimental design is shown in Table 29.

**Table 29. Experimental design overview**

| Groups | Substances administered | Route of administration | Number of animals (animal) |
|---|---|---|---|
| Control group | Normal saline | Intravitreal injection | 10 |
| IBI302 8 mg/animal group | IBI302 | Intravenous injection | 10 |
| IBI302 0.5 mg/eye group | IBI302 | Intravitreal injection | 10 |
| IBI302 2 mg/eye group | IBI302 | Intravitreal injection | 10 |
| IBI302 4 mg/eye group | IBI302 | Intravitreal injection | 10 |

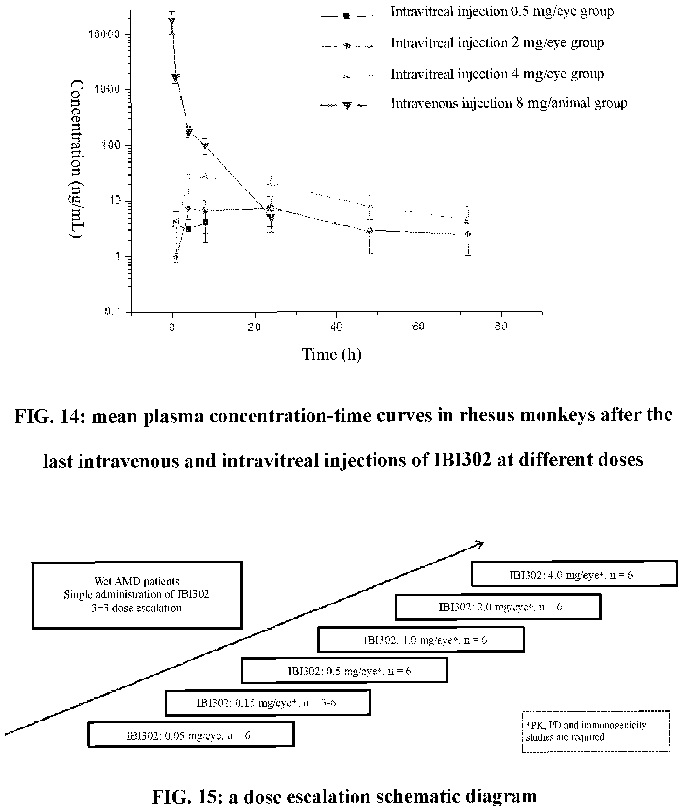

The results showed that the accumulation index of rhesus monkeys after continuous intravenous injections of IBI302 was 0.7. After the first administration of the repeated intravitreal injections of IBI302 at 0.5 mg/eye, 2 mg/eye and 4 mg/eye in rhesus monkeys, the Cₘₐₓ and the AUC₍₀₋ₜ₎ ratios were 1:4.4:5.5 and 1:6.3:13.4, respectively, indicating a certain dose-dependence relationship; after the last administration, the Cₘₐₓ and AUC₍₀₋ₜ₎ ratios were 1:2.3:7.9 and 1:15.7:54.6, respectively. The cumulative coefficients for the IBI302 dose groups were NA (no drug detected in individual animals after the last administration, unable to be calculated), 0.3 and 0.4, respectively. The specific results are shown in Tables 30 and 31 and FIGs. 13 and 14.

**Table 30. Toxicokinetic parameters in rhesus monkeys after the first intravenous and intravitreal injections of IBI302**

| Parameters | Unit | Intravenous injection 8 mg/animal group | Intravitreal injection 0.5 mg/eye group | Intravitreal injection 2 mg/eye group | Intravitreal injection 4 mg/eye group |
|---|---|---|---|---|---|
| AUC(0-t) | ng·h/mL | 24717.2±7939.8 | 193.5±95.9 | 1222±363.2 | 2593.7±707.1 |
| Cmax | ng/mL | 29133.8±9395.9 | 5.2±2.1 | 22.9±8.8 | 28.4±7.8 |
| Tmax | h | 0.1±0 | 11.2±9 | 12±14 | 24±21.6 |

**Table 31. Toxicokinetic parameters in rhesus monkeys after the last intravenous and intravitreal injections of IBI302**

| Parameters | Unit | Intravenous injection 8 mg/animal group | Intravitreal injection 0.5 mg/eye group | Intravitreal injection 2 mg/eye group | Intravitreal injection 4 mg/eye group |
|---|---|---|---|---|---|
| AUC(0-t) | ng·h/mL | 14789.7±4457.2 | 17.8±15.2 | 278.7±201.1 | 971.2±627.3 |
| Cmax | ng/mL | 17872.5±7692.7 | 4.1±2.5 | 9.4±4.2 | 32.3±17.1 |
| Tmax | h | 0.1±0 | 4.4±3.5 | 17.8±9.4 | 12±9.2 |
| Cumulative coefficient | | 0.7±0.4 | / | 0.3±0.3 | 0.4±0.4 |

The results indicated that after the repeated intravitreal injections of IBI302 in rhesus monkeys, the drug exposure and peak concentration increased with the increase of dose, showing a certain dose-dependence. The drug exposure in the serum of rhesus monkeys in all groups after the last injection was reduced to different degrees as compared to the drug exposure after the first administration. This, in combination with immunogenicity results, indicated that some of animals produce the anti-antibody and that no accumulation is observed after the repeated administrations.

### Distribution

### See pharmacokinetic study of IBI302 in rhesus monkeys of Example 1.

### Metabolism

According to "ICH S6 Preclinical Safety Evaluation of Biotechnology-Derived Pharmaceuticals" and "General Principles for the Evaluation of Non-Clinical Safety Technology for Biological Products for Treatment", IBI302 is an antibody drug and does not require metabolic studies, so that no study related to the metabolism of IBI302 was performed.

According to the metabolic rule of such drugs, the metabolites of IBI302 are expected to be peptide fragments or amino acids.

### Excretion

No study was performed.

### Conclusion

### Conclusion of pharmacokinetic study

After the intravitreal injection of IBI302 in rhesus monkeys, the metabolic rate of IBI302 is significantly lower in the local (ocular) area than in the whole body, and the local drug concentration is significantly higher in the eye than in the serum, suggesting that most of the drugs are limited in the eye, and the risk of systemic toxicity is lower. The drug exposure in the eye indicates that the drug is mostly distributed in the vitreous humor, retina and choroid to exert its pharmacological effect. After repeated intravitreal injections of IBI302 in rhesus monkeys, the drug exposure and peak concentration increased with the increase of dose, showing a certain dose-dependence. The drug exposure in the serum of rhesus monkeys in all groups after the last injection was reduced to different degrees as compared to the drug exposure after the first administration, indicating, in combination with immunogenicity results, that part of the animals produce the anti-antibody and that no accumulation is observed after the repeated administrations.

### Pharmacokinetic characteristics and significance

(1) Tissue distribution: after the intravitreal injection of IBI302, most of the drugs are limited in the eye, and the local drug concentration is significantly higher in the eye than in the serum, suggesting that IBI302 mainly exerts pharmacological activity in the eye, and the risk of systemic toxicity is lower. Meanwhile, the drug is mostly distributed in the vitreous humor, the retina and the choroid, which is in line with the design goal that the drug mainly acts on the retina and choroid.
(2) Long duration of efficacy: after the intravitreal injection of IBI302, the drug can remain in the retina and choroid for a long time whether in terms of the terminal elimination half-life T1/2 or the mean residence time MRT, which helps it to take effect.
(3) Accumulation: after repeated intravenous or vitreous injections of IBI302 in rhesus monkeys, the drug exposure is lower after the last administration than after the first administration, suggesting that the drug has no accumulation.
(4) Immunogenicity: after repeated intravenous or intravitreal injections of IBI302, the drug exposure in the serum of rhesus monkeys in all groups after the last injection is reduced to different degrees as compared to the drug exposure after the first administration, suggesting that part of the animals produce the antibody against IBI302.

### Example 4: Toxicologic Study

### Toxicity of single administration

After a single intravitreal injection of IBI302 at 2 mg/eye or 4 mg/eye, the rhesus monkeys showed no significant toxic responses after 14 days of observation, indicating that rhesus monkeys were well tolerated by the single intravitreal injection of IBI302 at 2 mg/eye or 4 mg/eye.

### Toxicity of repeated administrations

50 rhesus monkeys were randomly divided into the following 5 groups: a control group, an IBI302 intravenous injection 8 mg/monkey group, an IBI302 intravenous injection 0.5 mg/eye group, an IBI302 intravenous injection 2 mg/eye group, and an IBI302 intravenous injection 4 mg/eye group, with 10 animals in each group, half male and half female. The control group and the IBI302 4 mg/eye group were intravitreally injected with 0.9% sodium chloride injection and 40 mg/mL IBI302 at both eyes at 100 µL/eye, respectively, and the IBI302 0.5 mg/eye group and the IBI302 2 mg/eye group were intravitreally injected with 10 mg/mL IBI302 and 40 mg/mL IBI302 at both eyes at 50 µL/eye, respectively. The administration was performed once every 2 weeks for 3 consecutive times, with the recovery period after the interruption of 74 days. The specific experimental design is shown in Table 32.

**Table 32. Experimental design of toxicity study on repeated intravitreal injections of IBI302 in rhesus monkeys**

| Groups | Route of administration | Administration dosage (mg/eye) | Administration concentration (mg/mL) | Administration volume (µL/eye) | Number of animals (monkey) | |
|---|---|---|---|---|---|---|
| | | | | | Female | Male |
| Control group | Intravitreal injection | - | - | 100 | 5 | 5 |
| IBI302 intravenous injection group | Intravenous injection | 8 mg/monkey | 40 | 0.2 mL/monkey | 5 | 5 |
| IBI302 low-dose group | Intravitreal injection | 0.5 | 10 | 50 | 5 | 5 |
| IBI302 medium-dose group | Intravitreal injection | 2 | 40 | 50 | 5 | 5 |
| IBI302 high-dose group | Intravitreal injection | 4 | 40 | 100 | 5 | 5 |

### The results showed that:

There were no abnormal changes in the body weight, body temperature, food intake, hematology, serum chemistry, urine index, bone marrow index, ECG and blood pressure, organ weight and organ coefficient, histopathological examination of all organs except eyeballs and optic nerve, and the like of rhesus monkeys in the intravenous injection group and intravitreal injection groups.

Intravitreal injection of IBI302 at all doses resulted in endophthalmitis of different degrees in rhesus monkeys, which was mainly manifested as follows.

0.5 mg/eye group: during the treatment, in 1/10 of rhesus monkeys (beginning 3 days after the first administration in the right eye and 7 days after the second administration in the left eye), severe vitreous opacity/refractive medium opacity, fundus invisibility or white sheath-like changes in retinal branch veins, hemorrhagic foci, and vitreous opacity were observed by indirect ophthalmoscopy; the anterior chamber exudation, intraocular pressure reduction, vitreous opacity, unclear iris texture, aqueous flare, aqueous cells, corneal edema and opacity or anterior chamber exudation, AR and vitreous opacity were observed by the slit lamp examination; the intraocular pressure was significantly reduced; white blood cell count (WBC), monocytes (MONO), and neutrophils and percentages (NEU, NEU%) were significantly increased; and inflammatory cell infiltration and vitreous degeneration were observed by the pathological examination.

At week 4 of the recovery period, white sheath-like changes around the retinal branch vessels were observed in 1/4 of rhesus monkeys, and by week 6 of the recovery period, the rhesus monkeys were substantially recovered, and only slight inflammatory cell infiltration was observed in individual eyes by the pathological examination.

2 mg/eye group: during the treatment, in 1/10 of monkeys (beginning 3 days after the second administration in the left eye and 3 days after the third administration in the right eye), the anterior chamber exudation, severe vitreous opacity, fundus invisibility, or white sheath-like changes around the retinal branch veins with perivascular bleeding was observed by indirect ophthalmoscopy; the anterior chamber exudation, difficult pupil dilatation, chemosis, mixed congestion, corneal opacity, aqueous flare, unclear iris texture, or iris dropsy was observed by the slit lamp examination; the intraocular pressure was significantly reduced; WBC, MONO, NEU and NEU% were significantly increased; and the grayish-white color in the pupillary region of the anterior chamber of both eyeballs was observed by the gross anatomy. In another monkey, blood clots (possibly associated with the injection) appeared in the vitreous 1 day after the third administration, and vitreous opacity and fundus invisibility were observed by day 6 after the third administration. A decrease in the amplitude of the light adaptation 3.0 (Cone-R) b wave of female monkeys was observed at the end of the administration by the visual electrophysiology (ERG) examination; and monocyte infiltration, neutrophil infiltration, vitreous degeneration and retinal/iris dropsy in eyeballs and mild monocyte infiltration in the optic nerve were observed by the pathological examination.

At week 4 of the recovery period, white sheath-like changes around the retinal branch vessels were observed in 2/4 of rhesus monkeys, and by week 6 of the recovery period, the rhesus monkeys were substantially recovered; at weeks 4 and 7 of the recovery period, a decrease in the amplitude of the light adaption 3.0 (Cone-R) b wave in female monkeys was observed; only mild monocyte infiltration in the optic nerve, ciliary body or retina of individual eyes was observed by the pathological examination.

4 mg/eye group: during the treatment, in 1/10 of monkeys (both eyes, 6 days after the third administration), vitreous opacity was observed in both eyes by indirect ophthalmoscopy; and the anterior chamber exudation and vitreous opacity were observed by the slit lamp examination. In another monkey, the anterior chamber exudation in the left eye was observed 1 day after the first administration. A decrease in the amplitudes of the light adaption 3.0 (Cone-R) b wave and dark adaption 3.0 (Max-R) a wave of the female monkeys was observed at the end of the administration. The monocyte infiltration, vitreous degeneration and retinal edema in eyeballs and mild monocyte infiltration in the optic nerve were observed by the pathological examination.

At week 4 of the recovery period, white sheath-like changes around the retinal branch vessels were observed in 3/4 of monkeys by indirect ophthalmoscopy, and by week 10 of the recovery period, the rhesus monkeys were substantially recovered; and at week 5 of the recovery period, vitreous opacity was observed in the both eyes of 1 monkey (1/4) by the slit lamp examination, and by week 6 of the recovery period, the monkey was substantially recovered. A decrease in the amplitude of the light adaption 3.0 (Cone-R) b wave in female monkeys was observed at weeks 4 and 7 of the recovery period, but not observed at week 10 of the recovery period; in 2/4 of monkeys eyes, significant edema and thickening of retina around fundus blood vessels, enhanced reflection at retinal blood vessels, and dark shadow of corresponding tissues below the retina were observed by the OCT scanning at week 4 of the recovery period, but not observed at week 7 of the recovery period. Only mild monocyte infiltration in the optic nerve, ciliary body or retina of individual eyes was observed by the pathological examination.

In conclusion: after the intravitreal injection of IBI302 at 0.5 mg/eye, 2 mg/eye or 4 mg/eye, or the intravenous injection of IBI302 at 8 mg/animal in the rhesus monkeys once every 2 weeks for 3 consecutive times, with the recovery period of 74 days, no systemic toxicity was observed in all dose groups, and only endophthalmitis of individual animals was found; at the end of the recovery period, the endophthalmitis was substantially reversed in all groups by ophthalmic examinations, and significant reverse of histopathological changes in eyes was observed.

### Genetic toxicity

According to "ICH S6 Preclinical Safety Evaluation of Biotechnology-Derived Pharmaceuticals" and "General Principles for the Evaluation of Non-Clinical Safety Technology for Biological Products for Treatment", IBI302 is an antibody drug, and the metabolites are peptides or amino acids, which have no effect on DNA and other genetic materials, and do not require genotoxicity studies, so that no study related to the genetic toxicity of IBI302 was performed.

### Carcinogenicity

The product is intravitreally injected, has small systemic exposure, and is intended for an indication population over 50 years old, so that carcinogenicity research was not performed.

### Reproductive toxicity

The product is intravitreally injected, has small systemic exposure, and is intended for an indication population over 50 years old, with little safety risk of reproductive toxicity to be considered, so that reproductive research was not performed.

### Local tolerance

After the eye drop of IBI302 at 0.5 mg/eye or 2 mg/eye (concentration of 10 mg/mL or 40 mg/mL, respectively) once every week for consecutive 4 weeks, the eyes of Japanese white rabbits were not stimulated.

### Other toxicological tests

### Immunogenicity

50 rhesus monkeys were randomly divided into the following 5 groups: a control group, an IBI302 intravenous injection 8 mg/monkey group, an IBI302 intravenous injection 0.5 mg/eye group, an IBI302 intravenous injection 2 mg/eye group, and an IBI302 intravenous injection 4 mg/eye group, with 10 animals in each group, half male and half female. The administration was performed once every 2 weeks for 3 consecutive times, with the recovery period after the interruption of 74 days.

The immunogenicity study result indicated that after the last administration, the level of circulating immune complex (CIC) of the female and male monkeys in the IBI302 intravitreal injection 2 mg/eye and 4 mg/eye groups was increased, and at week 4 of the recovery period, the level of CIC of the female monkey and male monkeys in the IBI302 intravitreal injection 4 mg/eye group was also increased. Some of the animals in all dose groups produced anti-drug antibodies, and the result of bioactivity analysis showed that the some of the anti-drug antibodies may be neutralizing antibodies, indicating that IBI302 has certain immunogenicity.

### Immunotoxicity

50 rhesus monkeys were randomly divided into the following 5 groups: a control group, an IBI302 intravenous injection 8 mg/monkey group, an IBI302 intravenous injection 0.5 mg/eye group, an IBI302 intravenous injection 2 mg/eye group, and an IBI302 intravenous injection 4 mg/eye group, with 10 animals in each group, half male and half female. The administration was performed once every 2 weeks for 3 consecutive times, with the recovery period after the interruption of 74 days.

In the immunotoxicity study, the experimental results indicated that there were no abnormal changes in other immune indexes, namely immunoglobulins IgG, IgA, IgM and T lymphocyte subpopulations CD3+, CD4+ and CD8+ of the female and male monkeys in all IBI302 dose groups. At the end of the treatment period and the recovery period, there were no abnormal changes in the absolute weight and organ coefficient of spleen and thymus of monkeys in all IBI302 dose groups, and there were also no abnormal changes observed by the pathological examination, indicating that IBI302 has no immunotoxicity.

### Other tests

### Tissue cross-reactivity

The immunological cross-reactivity of IBI302 with normal human and cynomolgus monkey tissues was investigated by the conventional immunohistochemical method (one-step method). The experimental results showed that: (1) at concentrations of 2 µg/mL and 20 µg/mL, specific staining of biotinylated IBI302 (Biotin-IBI302) and biotinylated aflibercept (Biotin-ZALTRAP) was observed in recombinant human VEGF-A165 protein smears; specific staining of Biotin-IBI302 was observed in the human complement C4b protein smears, and no specific staining of Biotin-ZALTRAP was observed in the human complement C4b protein smears; in the negative control group, no specific staining of Biotin-IBI302 or Biotin-ZALTRAP was observed, and no staining was observed in all protein smears in the reagent control group (IBI302 placebo), indicating that the experimental system was established. (2) at concentrations of 2 µg/mL and 20 µg/mL, no specific immunohistochemical of IBI302 and aflibercept was observed in normal human and cynomolgus monkey tissues.

### Conclusion

### Conclusion of toxicology study

After a single intravitreal injection of IBI302 at 2 mg/eye or 4 mg/eye, the rhesus monkeys show no significant toxic responses and are well tolerated. After the intravitreal injection of IBI302 at 0.5 mg/eye, 2 mg/eye or 4 mg/eye, or the intravenous injection of IBI302 at 8 mg/animal in the rhesus monkeys once every 2 weeks for 3 consecutive times, with the recovery period of 74 days, the results show that no significant systemic toxicity is observed, and that the intravitreal injection of IBI302 at all doses results in endophthalmitis of different degrees in rhesus monkeys, and corresponding inflammatory pathological changes of the eye and optic nerve are observed. At the end of the recovery period, the endophthalmitis is substantially reversed in all groups by ophthalmic examinations, and significant reverse of histopathological changes in eyes is observed. The intravenous injection group and the intravitreal injection dose groups all can produce anti-drug antibodies, and some of the anti-drug antibodies are neutralizing antibodies that cannot be completely eliminated after 10 weeks of recovery. Endophthalmitis may be due to the anti-drug antibody production and immune complex formation, and may be less likely to occur in clinical studies, and therefore has a lower risk. Meanwhile, after the eye drop of IBI302 at 0.5 mg/eye or 2 mg/eye once every week for consecutive 4 weeks, the eyes of Japanese white rabbits are not stimulated. IBI302 has no significant tissue cross-reactivity with normal human and cynomolgus monkey tissues.

### Toxicological characteristics and significance

(1) Single administration: after a single intravitreal injection of IBI302 at 2 mg/eye or 4 mg/eye, the rhesus monkeys show no significant toxic responses, with the central nervous system, respiratory system and cardiovascular system not affected significantly, suggesting that the single intravitreal injection of IBI302 is highly safe.
(2) Multiple administrations: after the intravitreal injection of IBI302 at 0.5 mg/eye, 2 mg/eye or 4 mg/eye, or the intravenous injection of IBI302 at 8 mg/animal in the rhesus monkeys once every 2 weeks for 3 consecutive times, with the recovery period of 74 days, no obvious systemic toxicity is observed. The main symptoms are that endophthalmitis of different degrees is observed in intravitreal injection groups, and corresponding inflammatory pathological changes of the eye and optic nerve are observed. At the end of the recovery period, the endophthalmitis is substantially reversed in all groups by ophthalmic examinations, and significant reverse of histopathological changes in eyes is observed, suggesting that the toxic effects of IBI302 only occur locally in the eyes and are reversible, therefore IBI302 has good safety.
(3) Immunotoxicity and immunogenicity: after repeated intravitreal injections of IBI302 in rhesus monkeys, no immunotoxicity is observed. All IBI302 dose groups can produce anti-drug antibodies with higher incidence, suggesting that IBI302 has certain immunogenicity in rhesus monkeys, but it is still suggested to pay close attention in clinical studies.
(4) Endophthalmitis: the endophthalmitis after the intravitreal injection of IBI302 is associated with the anti-drug antibody production and immune complex formation, and may be less likely to occur in clinical studies considering that IBI302 is a fully human monoclonal antibody, and therefore has a lower risk, but it is still suggested to pay close attention in clinical studies.
(5) Local stimulation experiment: after the eye drop of IBI302 at 0.5 mg/eye or 2 mg/eye once every week for consecutive 4 weeks, the eyes of Japanese white rabbits are not stimulated, suggesting that IBI302 has no stimulation to the eyes.
(6) Tissue specificity: IBI302 has no significant cross-reactivity with normal human and cynomolgus monkey tissues, suggesting that IBI302 has no significant cross-reactivity with the normal human tissues and has very low target-related toxicity.

### Example 5: Dose-escalation Phase I clinical study of tolerance and safety of a single administration in patients with wet age-related macular degeneration (AMD)

### Purpose

To evaluate the safety, tolerance, bioactivity and efficacy of a single intravitreal injection of IBI302 in wet AMD patients.

### Drug

IBI302, specification: 0.2 mL: 2 mg; 0.2 mL: 8 mg.

The doses of 0.5 mg/eye, 2 mg/eye and 4 mg/eye can be directly extracted from the study drug of the current specifications.

Other doses were formulated as follows:
extracting a fixed volume of the study drug from a vial with a syringe;
injecting the fixed volume of the study drug into a vehicle bottle with corresponding volume;
pulling out the syringe, and mixing the vehicle and the study drug evenly; and
extracting 50 µL of the mixed solution from the vehicle bottle with a new syringe for intraocular injection.

The drug volume and corresponding vehicle volume to be extracted for each dose group are as follows:

| Dose group | Specifications | Drug volume to be extracted | Vehicle volume | Volume to be extracted (intraocular injection) |
|---|---|---|---|---|
| 0.05 mg/eye | 0.2 mL: 2 mg | 90 µL | 760 µL | 50 µL |
| 0.15 mg/eye | 0.2 mL: 8 mg | 60 µL | 760 µL | 50 µL |
| 0.5 mg/eye | 0.2 mL: 2 mg | 50 µL | - | 50 µL |
| 1 mg/eye | 0.2 mL: 8 mg | 760 µL | 760 µL | 50 µL |
| 2 mg/eye | 0.2 mL: 8 mg | 50 µL | - | 50 µL |
| 4 mg/eye | 0.2 mL: 8 mg | 100 µL | - | 100 µL |

### Study design

This is a multicenter, open-label, dose escalation study, and patients with active choroidal neovascularization (CNV) under the macular fovea secondary to wet AMD can be enrolled in this clinical study. Each subject should not have both eyes as a study eye. Eligible subjects will receive an intravitreal injection of the study drug on the first day of the study (D1). After the administration, the subjects should be monitored for safety in the study center until D2 and cannot leave the study center until they are confirmed to be free of any clinically significant abnormalities. The subjects should return to the hospital for follow-up visits on D3-D6, D8, D15, D29 and D43. See FIG. 15 for a dose escalation schematic diagram.

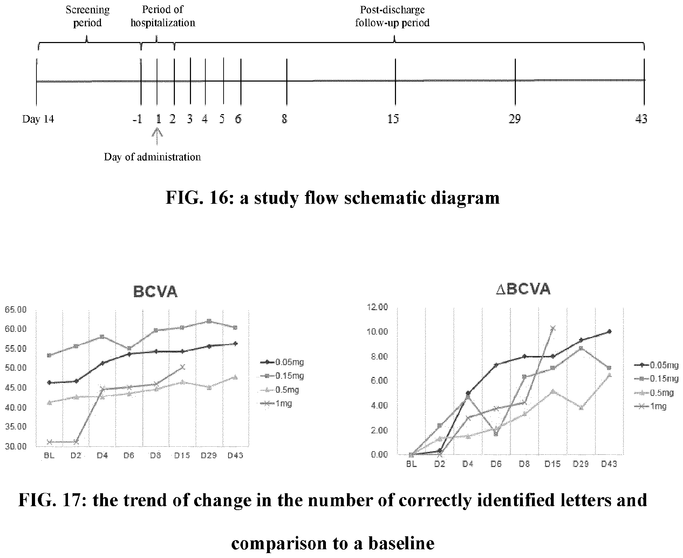

A total of 6 dose groups are set for this study: 0.05 mg/eye, 0.15 mg/eye, 0.5 mg/eye, 1 mg/eye, 2 mg/eye, and 4 mg/eye. A 3+3 dose escalation design is used. Dose-limiting toxicity (DLT) observed within 14 days after the administration (i.e., from post-dose on D1 to end of examinations on D15) is used as a basis for dose escalation. PK, PD and immunogenicity studies will be conducted in the 0.15 mg/eye to 4 mg/eye dose groups, with the dose groups assigned to be escalated to groups of 6 subjects before the maximum tolerated dose (MTD) after the completion of the dose escalation study in the 0.5 mg/eye to 4 mg/eye dose groups. See FIG. 16 for a study flow schematic diagram.

Dose-limiting toxicity is defined as any of the following IBI302-related adverse events (AEs) that occur during the DLT observation period:
mild or more severe endophthalmitis (see appendix 2);
intraocular pressure continuously increased by ≥ 10 mmHg for more than 60 min after the administration;
mild or more severe acute visual acuity decrease not related to endophthalmitis (see appendix 2);
moderate or more severe intravitreal hemorrhage (see appendix 2);
intracranial hemorrhage or other clinically significant extraocular hemorrhage; and
severe adverse events (SAEs) related to the study drug assessed by investigators.

### Appendix 2: Criteria for grading the expected adverse events of the eye

| **Grade** | **Criteria** |
|---|---|
| **Ocular inflammation:** | **defined as the presence of inflammatory cells that are active in the aqueous humor and/or vitreous humor, or visible hypopyon in the eye (excluding pigmented cells or apparently in the process of receding old inflammation). (Appendices 3 and 4)** |
| Severe | Any 4+ or 2-3+ ocular inflammation fails to decrease to 1+ or less within 30 days. |
| Moderate | Any 2+ ocular inflammation fails to decrease to 1+ or less within 14 days, or any 3+ ocular inflammation is decreased to 1+ or less within 30 days. |
| Mild | Any 2+ ocular inflammation is decreased to 1+ or less within 14 days. |

| **Visual acuity decrease:** (compared with visual acuity before injection) | |
|---|---|
| Severe | Visual acuity is decreased by ≥ 30 letters within 14 days after the injection of IBI302. |
| Moderate | Visual acuity is decreased by 21-29 letters within 14 days after the injection of IBI302; or visual acuity is decreased by 15-20 letters within 14 days after the injection of IBI302 without improvement by the next scheduled visit date. |
| Mild | The situation where visual acuity is decreased by 15-20 letters within 14 days after the injection of IBI302 is alleviated by the next scheduled visit date. |

| Intraocular pressure: (compared with intraocular pressure before injection) | |
|---|---|
| Severe | Light perception is lost persistently (> 15 min) due to increased intraocular pressure, or the situation where the intraocular pressure is increased or decreased |
| | by > 20 mmHg is maintained for more than 14 days. |
| Moderate | Intraocular pressure is increased or decreased by > 20 mmHg at any time within 24 h after the injection of IBI302, or intraocular pressure is increased or decreased by 10-20 mmHg for more than 14 days. |
| Mild | Intraocular pressure is increased or decreased by up to 10-20 mmHg at any time within 24 h after the injection of IBI302, and is alleviated or eliminated within 14 days. |

| **Vitreous hemorrhage: see "Grading of intravitreal hemorrhage degree" (appendix 4)** | |
|---|---|
| Severe | Vitreous hemorrhage ≥ 2+, cannot be eliminated within 14 days. |
| Moderate | Vitreous hemorrhage ≥ 2+, can be eliminated within 14 days. |
| Mild | Vitreous hemorrhage in trace amount or at 1+ |

| **Retinal tears or detachments** | |
|---|---|
| Severe | Retinal tears or detachments involving the macula occur during the study. |
| Moderate | Retinal tears or detachments not involving the macula occurs during the study. |
| Mild | None |

| **Retinal hemorrhage:** | |
|---|---|
| Severe | The new retinal hemorrhage is > 1 disc area and involves the macular fovea; or the original retinal hemorrhage area is increased by > 1 disc area and involves the macular fovea. |
| Moderate | The new retinal hemorrhage is > 1 disc area but does not involve the macular fovea; or the original retinal hemorrhage area is increased by > 1 disc area but does not involve the macular fovea. |
| Mild | The new retinal hemorrhage is ≤ 1 optic disc area; or the original retinal hemorrhage area is increased by ≤ 1 optic disc area. |

### Appendix 3: Criteria for grading the anterior chamber flare/cells

Reference is made to Hogan et al (Hogan MJ, Kimura SJ, Thygeson P.), Am J. Ophthalmol 1959; 47: 155-70, "Criteria for grading signs and symptoms of anterior uveitis and uveitis", the content of which is incorporated herein by reference in its entirety.

### Appendix 4: Criteria for grading intravitreal cell number and hemorrhage degree

| **Intravitreal cell number grading** | | |
|---|---|---|
| Cells in the retro illumination zone | Description | Grade |
| 0-1 | Clarity | 0 |
| 2-20 | Trace turbidity | Trace |
| 21-50 | Scattered turbidity | 1 |
| 51-100 | Moderate turbidity | 2 |
| 101-250 | Macro turbidity | 3 |
| >251 | Dense turbidity | 4 |

| **Grading of intravitreal hemorrhage degree** | | |
|---|---|---|
| None (0) | Retina is visible. | |
| Trace | Retina is visible with red blood cells. | |
| 1+ | Detailed structure of retina is visible with some hemorrhage. | |
| 2+ | General structure of retina is visible, but detailed structure of central retina is not visible. | |
| 3+ | Retinal red light reflection is visible, but structure of central retina behind the equator is not visible. | |
| 4+ | | Retinal red light reflection is absent. |

### Study population

Male or female aged 50 and above;
active CNV under the macular fovea secondary to wet AMD occurs in a study eye, with the size of lesion ≤ a diameter of 12 optic discs;
the best corrected visual acuity (BCVA) of the study eye is in the range of 10-73 letters as determined by the early treatment of diabetic retiopathy study (ETDRS) visual acuity scale;
central subfield thickness is ≥ 250 µm through optical coherence tomography (OCT) scanning;
the middle part and crystalline lens of the eye are not turbid, and clear stereoscopic fundus photography can be obtained;
female subjects of childbearing age or male subjects whose partners are women of childbearing age agree to take effective contraceptive measures from the beginning of the screening period to 3 months after the end of treatment; and
subjects voluntarily sign a written informed consent form and are able to comply with visits and related procedures specified in the protocol.

### Administration regimen

The subjects received a single intraocular injection of IBI302 at 0.05 mg/eye, 0.15 mg/eye, 0.5 mg/eye, 1 mg/eye, 2 mg/eye or 4 mg/eye, respectively, in an order of enrollment.

### Evaluation indexes

Safety evaluation indexes:
decrease in BCVA;
changes in intraocular pressure from a baseline after the administration;
incidence and severity of endophthalmitis;
changes in vital signs, physical examination results, and laboratory results from a baseline after the treatment; incidence of all adverse events (AEs), treatment emergent adverse events (TEAEs) and severe adverse events (SAEs), relevance to study drug, severity, and the like.

Efficacy evaluation indexes:
changes in central subfield thickness from a baseline on D8, D15, D29 and D43 by ophthalmic OCT examination;
CNV characteristics and changes in CNV area from a baseline on D43 by fluorescence angiography (FA) examinations;
changes in BCVA from a baseline on D2, D4, D6, D8, D15, D29 and D43.

Immunogenicity evaluation indexes:
Positive rate of the anti-drug antibody (ADA) and neutralizing antibody (NAb).

PK evaluation indexes:
PK parameters include, but are not limited to: area under the drug-time curve from time 0 to time t (AUCo-t), area under the drug-time curve from 0 to infinity (AUC_{0-∞}), peak concentration (Cₘₐₓ), time to peak (Tₘₐₓ), Clearance (CL), apparent volume of distribution (V), half-life (t_{1/2}), mean residence time (MRT), and the like.

PD evaluation indexes:
Free VEGF concentration, total VEGF concentration, complement-related indexes (C3, C4, C5 and cleaved fragments thereof), and inflammation-related indexes (IL-1b, IL-6).

### Statistical method

Sample size:
In this study, a total of 6 dose groups are set and the 6 dose levels are evaluated using a 3+3 dose escalation design, with the dose groups assigned to be escalated to groups of 6 subjects before MTD after the completion of the dose escalation study in the 0.5 mg/eye, 1 mg/eye, 2 mg/eye and 4 mg/eye dose groups, and a total of about 30-36 wet AMD patients are expected to be enrolled.

Statistical analysis:
Based on descriptive statistics, no comparisons between groups are made in principle. Safety, PK/PD parameters, immunogenicity, and efficacy indexes of a single intravitreal injection of IBI302 in wet AMD patients are summarized by dose groups.

The continuous variables are described using the number of cases, the mean, the standard deviation, the median, the minimum value and the maximum value; and the classification variables are described using frequency and percentage. In addition, for efficacy indexes, a 95% confidence interval for the mean value of the indexes for different dose groups will also be provided.

### Experimental results

Preliminary efficacy data for the treatment of IBI302 in nAMD were from Phase I single dose escalation study CIBI302A101 in a multicenter, open-label, dose escalation study design, with a total of 6 dose groups set: 0.05 mg/eye, 0.15 mg/eye, 0.5 mg/eye, 1 mg/eye, 2 mg/eye and 4 mg/eye, and eligible subjects received an intravitreal injection of the study drug on the first day of the study (D1). A 3+3 dose escalation design was used, with the dose groups assigned to be escalated to groups of 6 subjects before the maximum tolerated dose after the completion of the dose escalation study in the 0.5 mg/eye to 4 mg/eye dose groups. A total of 31 nAMD subjects were enrolled and received the study drug administration.

### 1. Safety and tolerance

There were no abnormalities in the following laboratory examinations: blood routine, serum chemistry, liver function and urine routine.

There were no abnormalities in vital signs or physical examinations, only 1 subject (1/15) had BCVA ↓ > 5 letters after the administration, which was returned to a baseline on day six after the administration; no SAE occurred, including endophthalmitis, arterial embolic events, retinal detachments, retinal pigment epithelium tears, and the most common AE was subconjunctival hemorrhage, (6/15, 40%, self-limiting, without affecting visual acuity), and two subjects had IOP > 21 mmHg (upper limit of normal values), which was self-limiting, and returned to normal level within 24 h.

### 2. Best corrected visual acuity (BCVA)

The test of the visual acuity of the subjects in the sitting position was started at the test distance of 4 meters using the ETDRS visual acuity scale. The number of correctly identified letters was counted and the results showed an increasing trend in the number of correctly identified letters. The initial test results are shown in FIG. 17, wherein the numerical value of BCVA represents the number of correctly identified letters; ΔBCVA% = AVERVGE (the number of correctly identified letters in the follow-up visit at this time - the number of correctly identified letters in the follow-up visit at the first time) × 100%. The mean value of each dose group is shown in the figure.

On days 2, 4, 6, 8, 15, 29 and 43 of the entire study, i.e., days 1, 3, 5, 7, 14, 28 and 42 after the administration, the mean best corrected visual acuity (BCVA) for the study eye of all subjects was improved by 2.1±3.87, 2.8±4.49, 3.8±4.69, 5.5±6.19, 5.9±6.86, 6.0±8.15, and 6.1±7.47 letters, respectively, from a baseline.

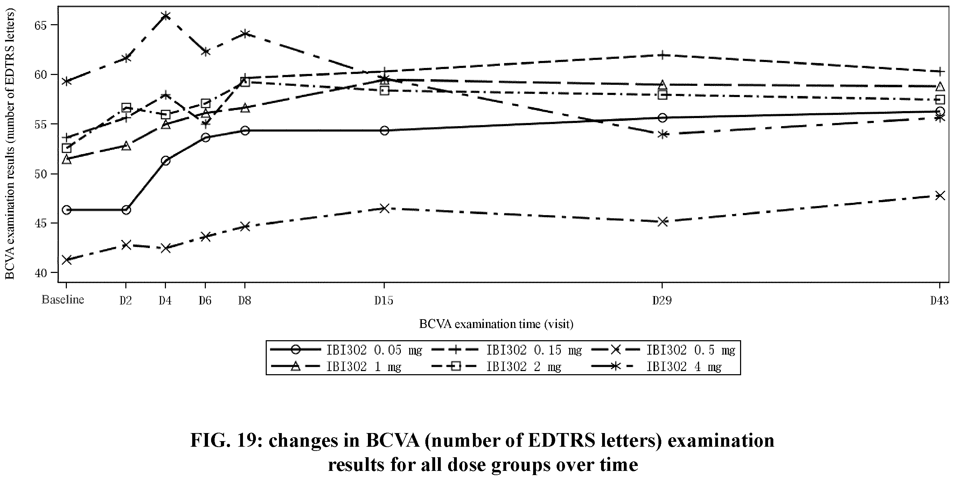

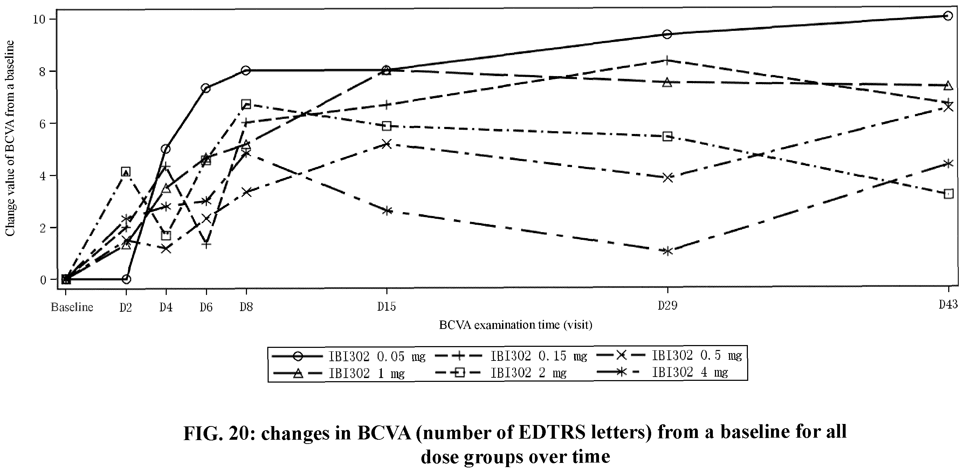

On days 2, 4, 6, 8, 15, 29 and 43, the BCVA for the study eye was improved in 83.9%, 74.2%, 87.1%, 83.9%, 87.1%, 77.4% and 71.0% of subjects, respectively, from a baseline, wherein the BCVA for the study eye was improved by ≥ 5 letters in 19.4%, 29.0%, 32.3%, 48.4%, 54.8%, 48.4% and 48.4% of the subjects, respectively; the BCVA for the study eye was improved by ≥ 10 letters in 6.5%, 3.2%, 16.1%, 22.6%, 25.8%, 25.8% and 22.6% of the subjects, respectively; and the BCVA for the study eye was improved by ≥ 15 letters in 0, 3.2%, 3.2%, 9.7%, 12.9%, 19.4% and 12.9% of the subjects, respectively. The mean values of BCVA examination results for the study eye of subjects in each dose group at each visit were improved from a baseline. The trend of change in the examination results over time and the trend of change in the change values of the examination results from a baseline over time are shown in FIG. 19 and FIG. 20, respectively. The mean value of each dose group is shown in the figure.

### 3. Optical coherence tomography (OCT) scanning

The OCT examination was performed in post-discharge follow-up visits on D8, D15, D29 and D43. After the administration, all subjects showed different degrees of absorption of intraretinal effusion (IRF) and subretinal effusion (SRF), and the absorption degree and the speed showed a certain dose-dependent relationship.

### 4. Central subfield thickness (CST)

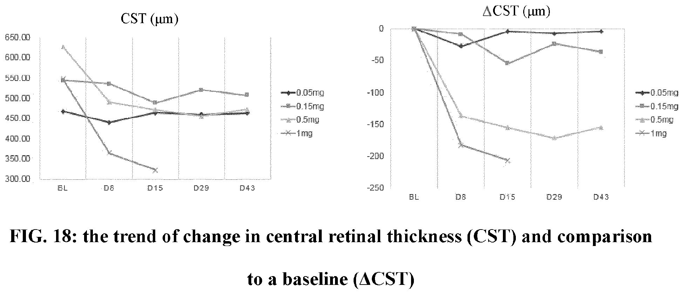

The central subfield thickness was examined in post-discharge follow-up visits on D8, D15, D29 and D43. After the administration, all subjects showed a decrease in the central subfield thickness, and the trend of thickness decrease showed a certain dose-dependent relationship. The trend of change in the central subfield thickness (CST) and initial test results as compared to a baseline are shown in FIG. 18. The mean value of each dose group is shown in the figure.

On days 8, 15, 29 and 43 of the entire study, i.e., days 7, 14, 28 and 42 after the administration, the mean reductions in CST of the study eye of all subjects by OCT examination were 100±80.62, 122.8±100.48, 141.2±153.66, and 66.1±205.05, respectively.

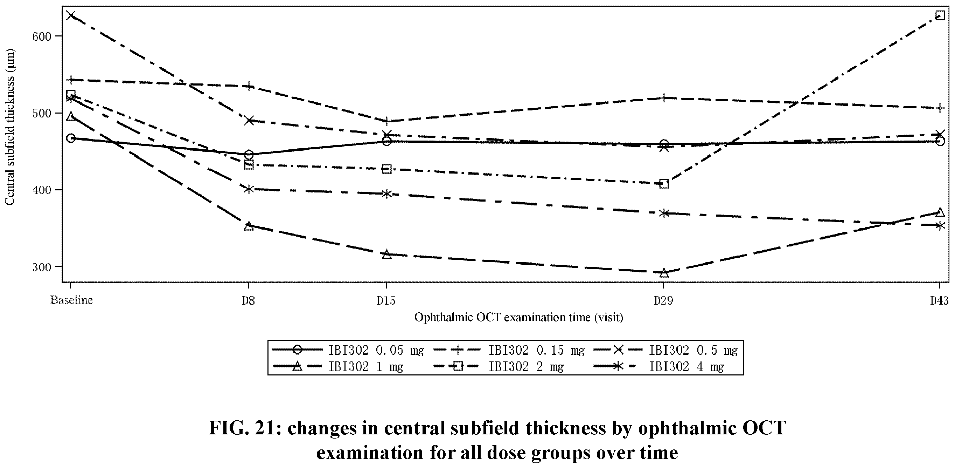

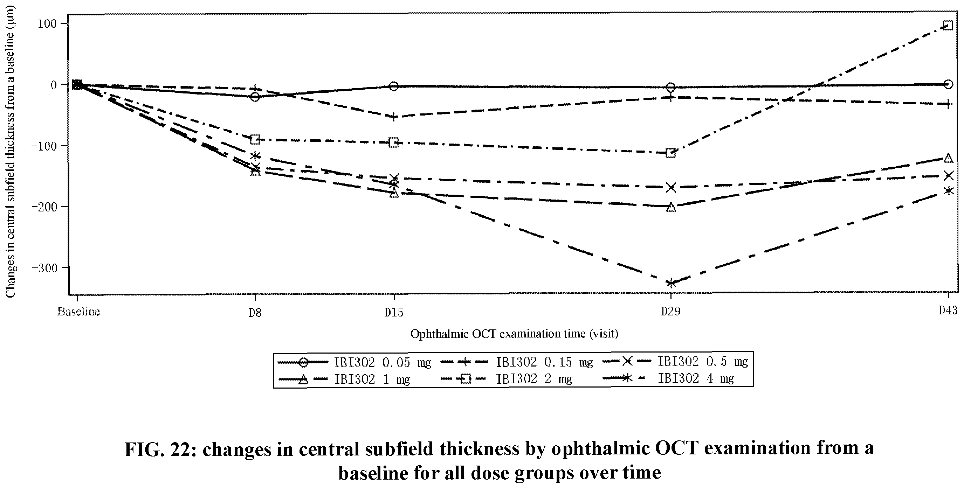

The CST of the study eye in all dose groups by OCT examination was reduced after the administration, and the degree of reduction in thickness reached the maximum on day 28 after the administration. Starting from the 0.5 mg dose group, the reduction in thickness was significantly higher than that of the 0.05 mg and 0.15 mg dose groups. The 4 mg dose group showed the greatest reduction at D29, up to 328.5 µm. The trend of change in the CST examination results for the study eye in all dose groups over time is shown in FIG. 21, and the trend of change in the changes in the CST examination results for the study eye in all dose groups from a baseline over time is shown in FIG. 22. The mean value of each dose group is shown in the figure.

Specifically, for the improvement of retinal thickness in the macula, the 1 mm central subfield thickness (CST) in the central region measured by OCT was used as an observation index. All dose groups showed a decrease in CST on day 7 after the administration. On day 7 after the administration (i.e., D8), the CST values of all dose groups (0.05 mg, 0.15 mg, 0.5 mg, 1 mg, 2 mg, and 4 mg) were decreased by 21.3±29.69 µm, 8.3±90.47 µm, 136.8±89.87 µm, 142±100.97 µm, 90.6±41.94 µm, and 117.5±44.70 µm from a baseline, respectively, with the percentages of decrease from a baseline of 5%, 2%, 22%, 29%, 17% and 23%, respectively. Among them, the CST improvement degree in the 0.5 mg and higher dose groups was significantly higher than that in the 0.05 mg and 0.15 mg dose groups. On day 28 after the administration (i.e., D29), the CST values of all dose groups (0.05 mg, 0.15 mg, 0.5 mg, 1 mg, 2 mg, and 4 mg) were decreased by 8.0±67.64 µm, 23.7±79.53 µm, 171.8±131.88 µm, 203.7±159.66 µm, 115.3±154.06 µm, and 328.5±191.63 µm from a baseline, respectively, with the percentages of decrease from a baseline of 1.7%, 4.4%, 27.4%, 41.1%, 22.0% and 63.3%, respectively. Among them, the CST improvement degree in the 0.5 mg and higher dose groups was significantly higher than that in the 0.05 mg and 0.15 mg dose groups. The effect of CST improvement continued until the end of the study.

### 5. Choroidal Neovascularization (CNV) area by FA examination

In the entire study, on day 42 after the administration, the mean reductions in CNV area of the study eye by FA examination were 0.524±3.7806 mm². The CNV area decreased in all dose groups except the 2 mg dose group on day 42 after the administration. The mean reduction in the CNV area of the contralateral eye was 0.352±1.7891 mm². On day 42 after the administration, the CNV area in the 0.05 mg dose group was decreased by 0.980±0.4857 mm² from a baseline, the CNV area in the 0.15 mg dose group was decreased by 4.963±9.9122 mm² from a baseline, the CNV area in the 0.5 mg dose group was decreased by 0.365±1.7140 mm² from a baseline, the CNV area in the 1 mg dose group was decreased by 0.820±1.7530 mm² from a baseline, and the CNV area in the 4 mg dose group was decreased by 0.327±0.4206 mm² from a baseline. The last non-missing value before the first administration of the study drug (including the data from the screening period) was used as a baseline for the above data. Change value = measured value - baseline value.

## Claims

1. A method for preventing or treating age-related macular degeneration (AMD) in an individual, comprising administering to the individual a fusion protein inhibiting a VEGF pathway and a complement pathway.

2. The method according to claim 1, wherein the fusion protein comprises an amino acid sequence of SEQ ID NO: 1.

3. The method according to claim 1, wherein the sequence of the fusion protein is a sequence set forth in SEQ ID NO: 1.

4. The method according to any one of claims 1-3, wherein the AMD is wet AMD.

5. The method according to claim 4, wherein the wet AMD has one or more of the following symptoms and signs: visual acuity decrease, metamorphopsia, central scotoma, dyslexia, macular retinal swelling, fundus hemorrhage, neovascularization, scar fibrosis, geographic atrophy, or any combination thereof.

6. The method according to claim 4, wherein the wet AMD has choroidal neovascularization (CNV).

7. The method according to claim 4, wherein the wet AMD is AMD having the following characteristics:
(1) active CNV under the macular fovea secondary to wet AMD occurs in a study eye; and/or
(2) central subfield thickness is ≥ 250 µm through optical coherence tomography (OCT) scanning.

8. The method according to any one of the above claims, wherein the individual is a human individual having the disease, symptoms, and/or characteristics according to any one of the above claims.

9. The method according to any one of the above claims, wherein the individual is a human individual who is a wet AMD patient accompanied by geographic atrophy.

10. The method according to any one of the above claims, wherein the individual suffers from wet AMD with the following characteristics:
(1) active CNV under the macular fovea secondary to wet AMD occurs in a study eye; and/or
(2) central subfield thickness is ≥ 250 µm through optical coherence tomography (OCT) scanning.

11. The method according to any one of the above claims, wherein the treatment can achieve one or more of the following effects in the individual:
1) central subfield thickness is decreased from a baseline;
2) area of the choroidal neovascularization (CNV) is reduced;
3) best corrected visual acuity (BCVA) is improved from a baseline;
4) onset of the geographic atrophy in the wet AMD patient is lagged; and/or
5) onset of the geographic atrophy in the wet AMD patient is slowed.

12. The method according to any one of the above claims, wherein the treatment can achieve one or more of the following effects in the individual:
1) best corrected visual acuity (BCVA) is improved; and/or
2) central subfield thickness is decreased; and/or
3) choroidal neovascularization (CNV) area is reduced.

13. The method according to any one of the above claims, wherein the treatment can achieve one or more of the following effects in the individual:
1) best corrected visual acuity (BCVA), as compared to a baseline, is improved by at least 1 ETDRS letter, preferably by at least 5 ETDRS letters, more preferably by 5-35 ETDRS letters, for example, by 5-25 ETDRS letters, for example, by 5-10, 5-15, 5-20, 10-15, 10-20, 10-25, 15-20 or 15-25 letters, for example, by 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 letters; and/or
2) best corrected visual acuity (BCVA), as compared to a baseline, is improved by 5% or more, preferably by 10% or more, for example, by 10%-200%, preferably by 10%-150%, more preferably by 10%-100%, more preferably by 20%-100%, more preferably by 50%-100%, 60%-100%, 20%-80% or 30%-70%, more preferably by 50%-70%, more preferably by 60%-70%, for example, by 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190% or 200%, and/or
3) central subfield thickness (CST), as compared to a baseline, is decreased by 25 µm or more, for example, by 25-350 µm, preferably by 50-300 µm, more preferably by 50-200 µm, for example, by 25 µm, 50 µm, 75 µm, 100 µm, 125 µm, 150 µm, 175 µm, 200 µm, 225 µm, 250 µm, 275 µm, 300 µm, 325 µm or 350 µm; or is decreased by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%; and/or
4) area of the choroidal neovascularization (CNV), as compared to a baseline, is reduced by 0.1 mm² or more, for example, by 0.1-20 mm², preferably by 0.2-5 mm², more preferably by 0.3-2 mm², for example, by 0.1 mm², 0.2 mm², 0.3 mm², 0.4 mm², 0.5 mm², 0.6 mm², 0.7 mm², 0.8 mm², 0.9 mm², 1 mm², 1.1 mm², 1.2 mm², 1.3 mm², 1.4 mm², 1.5 mm², 1.6 mm², 1.7 mm², 1.8 mm², 1.9 mm², 2 mm², 2.1 mm², 2.2 mm², 2.3 mm², 2.4 mm², 2.5 mm², 2.6 mm², 2.7 mm², 2.8 mm², 2.9 mm², 3 mm², 3.1 mm², 3.2 mm², 3.3 mm², 3.4 mm², 3.5 mm², 3.6 mm², 3.7 mm², 3.8 mm², 3.9 mm², 4 mm², 4.1 mm², 4.2 mm², 4.3 mm², 4.4 mm², 4.5 mm², 4.6 mm², 4.7 mm², 4.8 mm², 4.9 mm², 5 mm², 5.5 mm², 6 mm², 6.5 mm², 7 mm², 7.5 mm², 8 mm², 8.5 mm², 9 mm², 9.5 mm², 10 mm², 10.5 mm², 11 mm², 11.5 mm², 12 mm², 12.5 mm², 13 mm², 13.5 mm², 14 mm², 14.5 mm², 15 mm², 15.5 mm², 16 mm², 16.5 mm², 17 mm², 17.5 mm², 18 mm², 18.5 mm², 19 mm², 19.5 mm² or 20 mm²; or is reduced by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

14. The method according to any one of the above claims, comprising intravitreally or intravenously administering to the individual the fusion protein at a single dose of about 0.01-10 mg/eye, preferably 0.05-8 mg/eye, more preferably 0.05-6 mg/eye, more preferably 0.5-5 mg/eye, more preferably 1-5 mg/eye, more preferably 2-5 mg/eye once a day, once every two days, twice a week, once a week, once every two weeks, once every four weeks, once every five weeks or once every six weeks, wherein central subfield thickness of the individual six or eight weeks after administration, as compared to central subfield thickness of the individual before administration, is decreased by 25 µm or more, for example, by 25-350 µm, preferably by 50-300 µm, more preferably by 50-200 µm, for example, by 25 µm, 50 µm, 75 µm, 100 µm, 125 µm, 150 µm, 175 µm, 200 µm, 225 µm, 250 µm, 275 µm, 300 µm, 325 µm or 350 µm; or is decreased by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

15. The method according to any one of the above claims, comprising intravitreally or intravenously administering to the individual the fusion protein at a single dose of about 0.01-10 mg/eye, preferably 0.05-8 mg/eye, more preferably 0.05-6 mg/eye, more preferably 0.5-5 mg/eye, more preferably 1-5 mg/eye, more preferably 2-5 mg/eye once a day, once every two days, twice a week, once a week, once every two weeks, once every four weeks, once every five weeks or once every six weeks, wherein area of the choroidal neovascularization (CNV) of the individual six or eight weeks after administration, as compared to area of the choroidal neovascularization (CNV) of the individual before administration, is reduced by more than 0.1 mm², for example, by 0.1-20 mm², preferably by 0.2-5 mm², more preferably by 0.3-2 mm², for example, by 0.1 mm², 0.2 mm², 0.3 mm², 0.4 mm², 0.5 mm², 0.6 mm², 0.7 mm², 0.8 mm², 0.9 mm², 1 mm², 1.1 mm², 1.2 mm², 1.3 mm², 1.4 mm², 1.5 mm², 1.6 mm², 1.7 mm², 1.8 mm², 1.9 mm², 2 mm², 2.1 mm², 2.2 mm², 2.3 mm², 2.4 mm², 2.5 mm², 2.6 mm², 2.7 mm², 2.8 mm², 2.9 mm², 3 mm², 3.1 mm², 3.2 mm², 3.3 mm², 3.4 mm², 3.5 mm², 3.6 mm², 3.7 mm², 3.8 mm², 3.9 mm², 4 mm², 4.1 mm², 4.2 mm², 4.3 mm², 4.4 mm², 4.5 mm², 4.6 mm², 4.7 mm², 4.8 mm², 4.9 mm², 5 mm², 5.5 mm², 6 mm², 6.5 mm², 7 mm², 7.5 mm², 8 mm², 8.5 mm², 9 mm², 9.5 mm², 10 mm², 10.5 mm², 11 mm², 11.5 mm², 12 mm², 12.5 mm², 13 mm², 13.5 mm², 14 mm², 14.5 mm², 15 mm², 15.5 mm², 16 mm², 16.5 mm², 17 mm², 17.5 mm², 18 mm², 18.5 mm², 19 mm², 19.5 mm² or 20 mm²; or is reduced by 5% or more, for example, by 10%-70%, preferably by 10%-60%, more preferably by 30%-50%, more preferably by 40%-50%, for example, by 5%, 10%, 15%, 20%, 25%, 30%, 35%, 40%, 45%, 50%, 55%, 60%, 65% or 70%.

16. The method according to any one of the above claims, comprising intravitreally or intravenously administering to the individual the fusion protein at a single dose of about 0.01-10 mg/eye, preferably 0.05-8 mg/eye, more preferably 0.05-6 mg/eye, more preferably 0.5-5 mg/eye, more preferably 1-5 mg/eye, more preferably 2-5 mg/eye once a day, once every two days, twice a week, once a week, once every two weeks, once every four weeks, once every five weeks or once every six weeks, wherein best corrected visual acuity (BCVA) of the individual six or eight weeks after administration, as compared to best corrected visual acuity (BCVA) of the individual before administration, is improved by at least 1 ETDRS letter, preferably by at least 5 ETDRS letters, more preferably by 5-35 ETDRS letters, for example, by 5-25 ETDRS letters, for example, by 5-10, 5-15, 5-20, 10-15, 10-20, 10-25, 15-20 or 15-25 letters, for example, by 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 letters; or is improved by 5% or more, preferably by 10% or more, for example, by 10%-200%, preferably by 10%-150%, more preferably by 10%-100%, more preferably by 20%-100%, more preferably by 50%-100%, 60%-100%, 20%-80% or 30%-70%, more preferably by 50%-70%, more preferably by 60%-70%, for example, by 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190% or 200%.

17. Use of the fusion protein inhibiting a VEGF pathway and a complement pathway in the manufacture of a medicament for preventing or treating age-related macular degeneration (AMD) in an individual, wherein the fusion protein comprises a sequence of SEQ ID NO: 1.

18. The use according to claim 17, wherein the sequence of the fusion protein is a sequence set forth in SEQ ID NO: 1.

19. The use according to any one of claims 17-18, wherein the AMD is as defined in any one of claims 4-7.

20. The use according to any one of claims 17-19, wherein the individual is as defined in any one of claims 8-10.

21. A single pharmaceutical dosage unit, comprising a fusion protein inhibiting a VEGF pathway and a complement pathway, wherein the fusion protein comprises a sequence of SEQ ID NO: 1.

22. The single pharmaceutical dosage unit according to claim 21, wherein the sequence of the fusion protein is a sequence set forth in SEQ ID NO: 1.

23. The single pharmaceutical dosage unit according to claim 21 or 22, comprising the fusion protein at the following doses: 0.01-10 mg, preferably 0.05-8 mg, more preferably 0.05-6 mg, more preferably 0.5-5 mg, more preferably 1-5 mg, more preferably 2-5 mg, for example, 0.5-2 mg, 1-3 mg, 2-4 mg, 2-3 mg or 3-5 mg, for example, 0.05 mg, 0.1 mg, 0.15 mg, 0.2 mg, 0.25 mg, 0.3 mg, 0.35 mg, 0.4 mg, 0.45 mg, 0.5 mg, 0.55 mg, 0.6 mg, 0.65 mg, 0.7 mg, 0.75 mg, 0.8 mg, 0.85 mg, 0.9 mg, 0.95 mg, 1 mg, 1.1 mg, 1.2 mg, 1.3 mg, 1.4 mg, 1.5 mg, 1.6 mg, 1.7 mg, 1.8 mg, 1.9 mg, 2 mg, 2.1 mg, 2.2 mg, 2.3 mg, 2.4 mg, 2.5 mg, 2.6 mg, 2.7 mg, 2.8 mg, 2.9 mg, 3 mg, 3.1 mg, 3.2 mg, 3.3 mg, 3.4 mg, 3.5 mg, 3.6 mg, 3.7 mg, 3.8 mg, 3.9 mg, 4 mg, 4.1 mg, 4.2 mg, 4.3 mg, 4.4 mg, 4.5 mg, 4.6 mg, 4.7 mg, 4.8 mg, 4.9 mg, 5 mg, 5.1 mg, 5.2 mg, 5.3 mg, 5.4 mg, 5.5 mg, 5.6 mg, 5.7 mg, 5.8 mg, 5.9 mg, 6 mg, 6.1 mg, 6.2 mg, 6.3 mg, 6.4 mg, 6.5 mg, 6.6 mg, 6.7 mg, 6.8 mg, 6.9 mg, 7 mg, 7.1 mg, 7.2 mg, 7.3 mg, 7.4 mg, 7.5 mg, 7.6 mg, 7.7 mg, 7.8 mg, 7.9 mg, 8 mg, 8.5 mg, 9 mg, 9.5 mg or 10 mg, preferably 2 mg or 4 mg.

24. A pharmaceutical kit, comprising the fusion protein according to any one of claims 21-23.

25. Use of the single pharmaceutical dosage unit according to any one of claims 21-23 or the pharmaceutical kit according to claim 24 in the manufacture of a medicament for preventing or treating age-related macular degeneration (AMD).

26. The single pharmaceutical dosage unit according to any one of claims 21-23 or the pharmaceutical kit according to claim 24, for use in the prevention or treatment of age-related macular degeneration (AMD).
